(19) Europäisches Patentamt
European Patent Office
Office européen des brevets

(11) EP 2 482 077 A1

(12) **EUROPEAN PATENT APPLICATION**

(43) Date of publication:
**01.08.2012 Bulletin 2012/31**

(51) Int Cl.:
***G01N 33/68*** *(2006.01)*

(21) Application number: **11152509.3**

(22) Date of filing: **28.01.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Max-Planck-Gesellschaft zur Förderung der Wissenschaften e.V.**
  **80539 München (DE)**
- **Ninsar AgroSciences SL**
  **08028 Barcelona (ES)**
- **Gautier Semences**
  **13630 Eyragues (FR)**

(72) Inventors:
- **Van Der Hoorn, Renier A.**
  **50259 Pulheim-Freimersdorf (DE)**
- **Gu, Christian**
  **50829 Köln (DE)**
- **Kaschani, Farnusch**
  **50858 Köln (DE)**
- **Misas, Johana**
  **50859 Köln-Widdersdorf (DE)**
- **Richau, Kerstin**
  **50674 Köln (DE)**
- **Kolodziejek, Izabella**
  **50823 Köln (DE)**
- **Moquet, Frédéric**
  **13690 Graveson (FR)**
- **Malliart, Capucine**
  **84000 Avignon (FR)**
- **Berthet, Xavier**
  **08023 Barcelona (ES)**

(74) Representative: **Neuefeind, Regina**
**Maiwald Patentanwalts GmbH**
**Elisenhof**
**Elisenstrasse 3**
**80335 München (DE)**

(54) **Seed trait prediction by activity-based protein profiling**

(57) The present invention relates to a method of predicting a plant seed trait by determining the presence of a target protein in its active state in a protein sample derived from a plant seed or plant seed lot. In particular, the method comprises contacting the protein sample or the plant seed or plant seed lot with a chemical probe comprising a warhead being able to attach to an amino acid residue in or nearby an active site of the target protein under conditions allowing the formation of a conjugate of the target protein and the chemical probe, wherein the chemical probe further comprises a reporter tag which is used to detect the conjugate, and wherein detection of the conjugate indicates the presence of the target protein in the protein sample and is used to predict the plant seed trait. The present invention further relates to the use of such chemical probes for the prediction of plant seed traits.

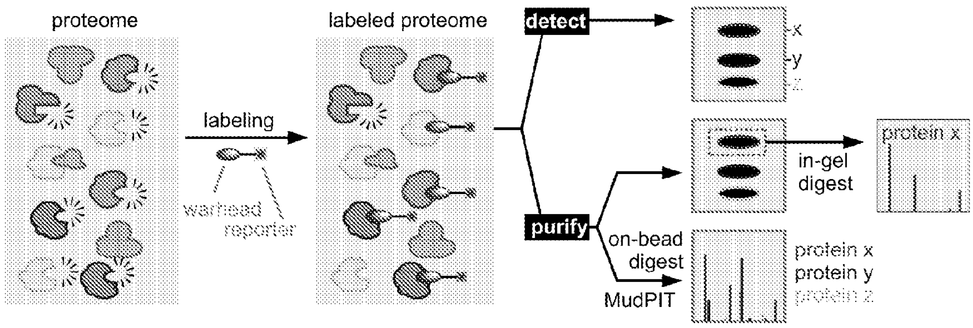


Figure 1



Printed by Jouve, 75001 PARIS (FR)

## Description

## SUBJECT OF THE INVENTION

The present invention relates to a method of predicting a plant seed trait by determining the presence of a target protein in its active state in a protein sample derived from a plant seed or plant seed lot. In particular, the method comprises contacting the protein sample or the plant seed or plant seed lot with a chemical probe comprising a warhead being able to attach to an amino acid residue in or nearby an active site of the target protein under conditions allowing the formation of a conjugate of the target protein and the chemical probe, wherein the chemical probe further comprises a reporter tag which is used to detect the conjugate, and wherein detection of the conjugate indicates the presence of the target protein in the protein sample and is used to predict the plant seed trait. The present invention further relates to the use of such chemical probes for the prediction of plant seed traits.

## BACKGROUND OF THE INVENTION

Determination of the genomic sequence of different organisms is an attainable goal. However, this analysis represents only one aspect of the information encoded by the genome. Accordingly, knowing the sequence of the genome is insufficient to explain biology. With the post-genome era rapidly approaching, new strategies for the analysis of proteins are being developed. Most conventional approaches focus on recording variations in protein-level, structure and composition. These approaches are commonly referred to as "proteomics". In general, proteomics seeks to measure the abundance of broad profiles of proteins from complex biological mixtures.

In particular, transcription of DNA is an early step in an extended process resulting ultimately in the expression of genomic information as a functional protein. Additional steps can include processing of the initial transcript to mRNA, translation of the mRNA into protein, and posttranslational processing of the protein (e.g., cleavage of the protein into smaller fragments, modification of the protein by glycosylation, methylation, acylation, phosphorylation, etc.). In addition, the protein may be activated or deactivated by interaction with other proteins and/or with small molecules (e.g., cofactors). Regulation of active protein expression can occur at one or more of these steps, and the amount of active protein in the cell at any time will vary widely with the state of the protein in the cell. Thus, the presence of a given gene in a cell's genome, or the total amount of a particular protein in a cell is not necessarily a good prognosticator of the state of the cell, as reflected by the amount of active protein present in the cell. Hence, neither genomic nor proteomic methods can selectively detect active versus inactive proteins within a sample.

A major challenge in plant science and biotechnology in general was thus directed to the provision of new tools to display the activities of proteins in order to understand how proteins act in an extract or living organism. Although current proteomic approaches could theoretically provide information on the abundance of an enzyme, these methods fail to report on enzyme activity. This is a key limitation because the activity of enzymes, and even other proteins, is often regulated by post-translational modification. Importantly, the active site of an enzyme represents only a small portion of the entire surface of the enzyme and the chemical nature and reactivity of this active site is governed by the local environment of the site, which is conferred by its amino acid compositions and its three dimensional structure. In particular, the shape and/or exposure of the active site of an enzyme can be modulated by any number of biological events.

Activity-based proteomics, or activity-based protein profiling (ABPP) is a novel functional proteomic technology that displays protein activities in proteomes. The technology was launched one decade ago by medicinal chemical biologists, mainly the laboratories of Dr. Cravatt (Scripps Institute, San Diego, CA, USA) and Dr. Bogyo (Stanford Medical School, CA, USA). ABPP is based on the use of labeled small reactive molecules (chemical probes) that react with active site residues of proteins, mostly in an activity-based manner. The reaction usually results in a covalent, irreversible bond between the protein and the probe, which facilitates subsequent analysis. Labeled proteins can be detected on protein gels or blots and purified and identified by mass spectrometry (MS).

The basic unit of ABPP is the chemical probe which typically consists of three elements: a reactive group, a linker and a tag. The reactive group usually contains an electrophile that gets covalently-linked to a nucleophilic residue in the active site of a protein, e.g. an active enzyme. An enzyme that is inhibited by enzyme inhibitors or post-translational modifications will generally not react with such an activity-based probe. Hence, a major advantage of ABPP is the ability to monitor the availability of the enzyme active site directly, rather than being limited to protein or mRNA abundance. This technique thus offers a valuable advantage over traditional techniques that rely on protein abundance rather than activity.

Accordingly, numerous applications of activity-based probes are known in the art, in particular to the screening of enzyme inhibitors, and the imaging of enzymes implicated in cancer. Some of these applications have been reviewed by D.A. Jeffry and M. Bogyo, Current Opinion in Biotechnology 2003, 14: 87-95, which discusses the use of synthetic small molecules to covalently modify a set of related enzymes and subsequently allow their purification and/or identification as valid drug targets as well as the application of activity-based protein profiling to drug screening and drug efficacy

studies; and B.F. Cravatt et al., Annual Review of Biochemistry 2008, 77: 383-414, which reviews the basic technology of activity-based protein profiling, the enzyme classes addressable by this method, and the biological discoveries attributable to its application.

In the prior art, several solutions have been sought for carrying out activity-based protein profiling, in particular in the field of cancer biology. Tumor cells are known to have elevated levels of hydrolytic and proteolytic enzymes, presumably to aid in key processes such as angiogenesis, cancer cell invasion, and metastasis. Hence, ABPP allows for the direct monitoring of enzyme activity in a physiological environment to better understand the roles of specific players in the complex process of tumorigenesis.

Accordingly, there was a strong demand for introducing ABPP technology to plant science. So far, early proof-of-concept studies have demonstrated the versatility and robustness of ABPP to study plant enzymes. These first biological studies of ABPP in plants have been reviewed by I. Kolodziejek and R.A.L. van der Hoorn (Current Opinion in Biotechnology 2010, 21:225-233), illustrating the potential of ABPP as a discovery tool to detect differential protein activities and inhibitors in plants. However, so far these studies were limited to plant proteomics, in particular to the study of senescence and plant-pathogen interactions. A major challenge in plant science is thus directed to the provision of novel ABPP applications.

The prediction of seed traits such as germination, dormancy, longevity, viability and vigour are important for agriculture since it is for these traits that farmers pay, and misprediction of seed traits can result in significant damage claims to the seed industry. However, seed traits are very difficult to predict since they are not only encoded in the genetic material, but also dependent on numerous factors which intervene throughout the seed life, from its development on the mother plant up to sowing (e.g., how the seeds were handled, harvested, stored, etc.).

Hence, seed quality is determined by a number of genetic and physiological characteristics. The genetic component involves differences between two or more genetic lines, while differences between seed lots of a single genetic line comprise the physiological component. The genetic factors that can influence quality include: genetic make-up, seed size, and bulk density. The physical or environmental characteristics include: injury during planting and establishment, growing conditions during seed development, nutrition of the mother plant, physical damage during production or storage by either machine or pest, moisture and temperature during storage and age or maturity of seed. Deterioration in seed quality may thus begin at any point in the plant's development stage from fertilization onward and seed quality depends upon the physical conditions that the mother plant is exposed during its growth stages, as well as harvesting, processing, storage and planting. Furthermore, temperature, nutrients and other environmental factors also affect seed development and influence seed quality.

High quality seeds are the result of good production practices, which include: proper maintenance of genetic purity, good growing conditions, proper timing and methods of harvesting, appropriate processing during threshing, cleaning and drying, as well as appropriate seed storage and seed distribution systems. A seed population is therefore heterogeneous, which leads to a lack of uniform performance. Thus, in the seed industry, high seed quality seeds are seeds that germinate completely, germinate quickly and simultaneously, produce normal seedlings and have germination which shows little sensitivity to environmental factors enabling them to germinate in a wide range of agro-climatic conditions.

Currently, many seed quality test assays rely on germination, followed by a phenotypic analysis. However, these methods are time-consuming and often too slow in the seed-trading season. Furthermore, one germination test is not sufficient to evaluate all seed quality characteristics. That's why seed companies very often do several tests to predict precisely the quality of the seeds they are selling. It was therefore an object of the present invention to provide a quick and reliable prediction of plant seed traits, in particular in high-value seeds such as tomato seeds.

## OBJECT AND SUMMARY OF THE INVENTION

It is an object of the present invention to provide a cheap, easy-to-use and reproducible bioassay which facilitates the prediction of plant seed quality, in particular the prediction of a plant seed trait. Accordingly, it is a further object of the present invention to provide a method capable of displaying several differential signals in single plant seeds, which signals segregate with plant seed traits, such as viability and germination. Furthermore, it would be desirable to identify more molecular markers that facilitate the prediction of plant seed traits.

According to an embodiment of the present invention, an activity-based protein profiling (ABPP) method for the prediction of seed traits in plant seeds, e.g. in tomato seeds, is provided. It has been found that ABPP can be used to screen seed proteomes with different chemical probes to identify molecular marker proteins that predict various seed traits. Such identified molecular markers can be tested for their robustness on different seed lots and the molecular origin of the markers can be identified by mass spectrometry. It has also been found that ABPP can be performed reproducible on single plant seeds, e.g. tomato seeds. Finally, it has been found that ABPP can display several differential signals that segregate with economically important seed traits such as viability and germination.

It has been surprisingly found that the inventive method offers a series of advantages compared to the traditional

assays. Firstly, the target protein does not need to be purified to distinguish it from other protein activities. This enables the target protein activity to be monitored in a more natural environment. Secondly, multiple enzyme activities can be monitored simultaneously, since one probe can label multiple family members at the same time. These different protein activities can be discriminated by separation on protein gels. Thirdly, it is not required to know a natural or artificial substrate of the protein. For many protein assays, finding substrates is still a bottleneck. Fourthly, ABPP is simple and robust and can be adjusted to high-throughput screening, enabling any lab with standard protein equipment to monitor protein activities by ABPP, e.g. by using automated extraction procedures and fluorescent probes.

These objectives as well as others which will become apparent from the ensuing description are attained by the subject matter of the independent claims. Some of the preferred embodiments of the present invention are defined by the subject matter of the dependent claims.

In one embodiment, the present invention relates to a method of predicting a plant seed trait by determining the presence of a target protein in its active state in a protein sample derived from a plant seed or plant seed lot, the method comprising:

(i) providing the protein sample;
(ii) contacting the protein sample or the plant seed or plant seed lot with a chemical probe under conditions allowing the formation of a conjugate of the target protein and the chemical probe, wherein the chemical probe comprises a warhead being able to attach to an amino acid residue in or nearby an active site of the target protein, and further comprises a reporter tag and a linker between the warhead and the reporter tag, and
(iii) using the reporter tag to detect the conjugate of (ii), wherein detection of the conjugate indicates the presence of the target protein in its active state in the protein sample and is used to predict the plant seed trait.

Preferably, the method further comprises separating the proteins contained in the protein sample by one or more means selected from the group consisting of liquid chromatography, electrophoretic separation, precipitation, immunocapture, microfluidic separation and mass spectrometry.

Also preferably, the conjugate is separated from non-target proteins by means of liquid chromatography, electrophoretic separation and/or mass spectrometry.

In a preferred embodiment, the method further comprises identifying the target protein by means of mass spectrometry.

In a further preferred embodiment, the target protein in its active state is an enzyme or a receptor in its active form.

Preferably, the protein sample is a protein extraction from a plant seed or a plant seed lot.

In a preferred embodiment of the present invention, the protein sample is provided by grinding at least one plant seed in an extraction buffer and removing insoluble materials by centrifugation.

Also preferably, the plant seed is derived from a vegetable or a field crop.

In a preferred embodiment of the present invention, the plant seed is derived from a plant selected from the group consisting of: Tomato (*Solanum lycopersicum*), Melon (*Cucumis melo*), Pepper (*Capsicum annuum*), Lettuce (*Lactuca sativa*), Eggplant (*Solanum melongena*), Cucumber (*Cucumis sativus*), Squash (*Cucurbita pepo*), Watermelon *(Citrullus lanatus),* Corn *(Zea mais),* Canola *(Brassica napus),* Sugar beet (*Beta vulgaris*), Potato (*Solanum tuberosum*), Wheat (*Triticum spp.*), Barley (*Hordeaum spp.*), and Cotton (*Gossypium hirsutum*).

In another preferred embodiment of the present invention, the warhead of the chemical probe comprises a reactive group directed to the active site of the target protein and an optional binding group, wherein

(i) the reactive group comprises an electrophilic atom that is susceptible to attack by an active-site nucleophilic residue of the target protein;
(ii) upon reaction of an active-site nucleophilic residue of the target protein with the reactive group, an electrophilic atom in the reactive group is provided, which electrophilic atom is susceptible to attack by a non-catalytic nucleophilic residue of the target protein;
(iii) upon addition of chemicals or UV light, a reactive center comprised by the reactive group is activated, which reactive center is susceptible to attack by an active-site nucleophilic or electrophilic residue of the target protein; or
(iv) the reactive group is a non-directed probe.

Preferably, the warhead of the chemical probe comprises a reactive group which is selected from the group consisting of epoxides, fluorophosphonases, acyloxymethylketones, epoxyketone, vinyl sulphone, and acylphosphates.

Also preferably, the reporter tag of the chemical probe is a detectable label selected from the group consisting of fluorescent moieties, radioactive moieties, electrochemical moieties, affinity moieties, and reactive moieties.

In a preferred embodiment of the present invention, the conditions allowing the formation of a conjugate of the target protein and the chemical probe comprise contacting the protein sample with the chemical probe at a pH of between 4.0 and 9.0.

In a further preferred embodiment of the present invention, the plant seed trait is selected from the group consisting of seed development, maturation, germination, germination rate, dormancy, storage deterioration, seed viability, sanitary state and vigour.

In a further embodiment, the present invention relates to the use of a chemical probe for the prediction of a plant seed trait, wherein the chemical probe comprises a warhead being able to attach to an amino acid residue in or nearby an active site of the target protein, and further comprises a reporter tag and a linker between the warhead and the reporter tag.

**FIGURE LEGENDS**

The accompanying drawings, which are incorporated and form part of the specification, merely illustrate certain preferred embodiments of the present invention. They are meant to serve to explain preferred modes of predicting plant seed traits to those of skilled in the art. In the drawings:

Figure 1 depicts a schematic overview of a preferred embodiment of the invention. A general workflow of the inventive method is shown. After providing the protein sample comprising a proteome (i.e. a complex mixture of proteins obtained from a plant seed), the proteome is contacted with a chemical probe comprising a warhead and a reporter tag under conditions allowing the formation of conjugates of target proteins and the chemical probe (i.e. the labeling reaction). Alternatively, the plant seed or plant seed lot can be directly contacted with the chemical probe and the protein sample comprising the proteome is subsequently provided. The chemical probe comprises a warhead comprising a reactive group, i.e. the portion of the chemical probe that can be directed to and binds to particular target proteins comprised by the proteome. Preferably, said binding is a covalent binding. As can be gathered from the second picture, the labeled proteome contains a number of unlabeled proteins as well as some labeled proteins (i.e. proteins x, y and z). Following the labeling reaction, the reporter tag comprised by the chemical probe can be used to directly detect the conjugates (third picture in the top), wherein detection of one of the conjugates indicates the presence of the respective target protein in its active state in the protein sample (i.e. proteins x, y or z). Since the reporter tag portion further permits capture of the conjugates of target proteins and the chemical probe, the target proteins may be further purified to identify the specific proteins to which the probes bound (e.g., isolated target proteins can be optionally characterized by mass spectrometric (MS) techniques). In particular, the sequence of isolated target proteins can be determined using tandem mass spectrometric techniques, and by application of sequence database searching techniques, the target protein from which a sequenced peptide originated can be identified. The third pictures in the middle and in the bottom depict in-gel digestion and identification of a particular target protein as well as on-bead digestion and identification of probe-labeled target proteins using the MudPIT technology.

Figure 2 depicts an illustration of the application of a preferred embodiment of the invention. A general workflow of a probe analysis in parallel by using fluorescence gel electrophoresis is shown. A plant seed extract was labeled using four different chemical probes. Labeling was performed on seed proteins in two buffers:

A: 50 mM NaOAc, pH 5.5, 1 mM DTT
B: 50 mM Tris, pH 7.6, 1 mM DTT.

Four different chemical probes have been used to label the plant seed extract:

1: VPE-R (Vacuolar Processing Enzyme)
2: ßN3DCG-04 (Cys proteases)
3: MV 151 (proteasome) and
4: FP-Rh (Ser hydrolases).

The labeled protein samples were then used for the preparation of a fluorescence gel. Control lanes (-) depict unlabeled protein extract. Robust labelling was detected with probes 2,3 and 4.

Figure 3 depicts a fluorescence gel demonstrating that ABPP can be used to label different varieties and different seed lots. Several plant seed extracts derived from different seed lots were labeled using the chemical probe FP-Rh (Ser hydrolases). Labeling was performed on seed proteins derived from seed lots A1, A3, B1 and B3. The labeled protein samples were then used for the preparation of a fluorescence gel. Control lanes (-) depict unlabeled protein extract. Robust labelling was detected for all seed lots.

Figure 4 depicts a fluorescence gel demonstrating that differential protein activities can be detected upon seed hydratation. A plant seed extract derived from a dry seed (lane D) and a plant seed extract derived from a seed which was hydrated for 24 hours (lane I) was labeled using three different chemical probes. Labeling was performed on seed proteins in two buffers:

A: 50 mM NaOAc, pH 5.5, 1 mM DTT
B: 50 mM Tris, pH 7.6, 1 mM DTT.

Three different chemical probes have been used to label the plant seed extracts, respectively:

1: ßN3DCG-04 (Cys proteases)
2: MV 151 (proteasome) and
3: FP-Rh (Ser hydrolases).

The labeled protein samples were then used for the preparation of a fluorescence gel. Control lanes (-) depict unlabeled protein extract. The last lane depicts an additional 40 kDa Ser hydrolase activity upon hydratation.

Figure 5 depicts a fluorescence gel demonstrating that the inventive method can be used to detect differences in enzyme activity between viable and non-viable seeds. Plant seed extracts derived from dead seeds (seed numbers 6, 24 and 41) and plant seed extracts derived from viable seeds (seed numbers 7, 14 and 35) were labeled using the chemical probe FP-Rh (Ser hydrolases). A further plant seed extract (seed number 13) derived from a seed which could not be classified as viable or non-viable. Control lanes (-) depict unlabeled protein extract. The labeled protein samples were then used for the preparation of a fluorescence gel. The lanes containing labeled protein extract depict an additional 53 kDa Ser hydrolase activity that correlates with seed viability.

Figure 6 depicts the chemical structures of the probes VPE-R, betaN3-DCG04, MV 151 and FP-Rh.

## DETAILED DESCRIPTION OF THE INVENTION

The present invention is based on the unexpected finding that ABPP is a cheap, quick, easy-to-use and reproducible bioassay which facilitates the prediction of seed traits, e.g. in tomato seeds. The method according to the present invention is capable of displaying several differential signals in single plant seeds, e.g. in single tomato seeds, which signals, *inter alia*, segregate with viability and/or germination. Furthermore, the inventive method can be used to identify molecular marker proteins targeted by chemical probes. It has been surprisingly found that such chemical probes can be used for the prediction of seed quality, i.e. for the prediction of plant seed traits.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of $\pm$ 10%, and preferably $\pm$ 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

In one embodiment, the present invention relates to a method of predicting a plant seed trait by determining the presence of a target protein in its active state in a protein sample derived from a plant seed or plant seed lot, the method comprising:

(i) providing the protein sample;
(ii) contacting the protein sample or the plant seed or plant seed lot with a chemical probe under conditions allowing the formation of a conjugate of the target protein and the chemical probe, wherein the chemical probe comprises a warhead being able to attach to an amino acid residue in or nearby an active site of the target protein, and further comprises a reporter tag and a linker between the warhead and the reporter tag, and
(iii) using the reporter tag to detect the conjugate of step (ii), wherein detection of the conjugate indicates the presence of the target protein in its active state in the protein sample and is used to predict the plant seed trait.

The present invention employs "chemical probes" that allow for the selective detection and optionally subsequent isolation of active target proteins from protein samples derived from plant seeds. The isolated target proteins are characteristic of the presence of a protein function, e.g., an enzymatic activity, protein complex formation, protein-nucleic acid interactions, etc., in those mixtures, and the presence of an active target protein in a protein sample can thus indicate a plant seed trait.

The "chemical probes" as used herein are chemical reagents that are polyfunctional molecules that substantially irreversibly bind to a target protein and generally inhibit the action of the target protein. The probes comprise at least a reactive functionality (i.e. the warhead) and a reporter tag, and have an affinity for a related group of proteins, whereby the warhead will bind to the target protein and can substantially inactivate the protein, and the reporter tag will permit detection and/or isolation. In referring to "affinity of a chemical probe for a target protein", one is concerned with the on-rate of the probe with the target protein, since there is generally no off-rate (the probe substantially irreversibly binds to the target protein).

Hence, the term "to attach to an amino acid residue in or nearby an active site of the target protein" as used herein denotes any mode of attachment, wherein the probe substantially irreversibly binds to the target protein, and preferably denotes a covalent attachment.

In a preferred embodiment, the present invention thus relates to a method of predicting a plant seed trait by determining the presence of a target protein in its active state in a protein sample derived from a plant seed or plant seed lot, the method comprising:

(i) providing the protein sample;
(ii) contacting the protein sample or the plant seed or plant seed lot with a chemical probe under conditions allowing the formation of a conjugate of the target protein and the chemical probe, wherein the chemical probe comprises a warhead being able to covalently attach to an amino acid residue in or nearby an active site of the target protein, and further comprises a reporter tag and a linker between the warhead and the reporter tag, and
(iii) using the reporter tag to detect the conjugate of step (ii), wherein detection of the conjugate indicates the presence of the target protein in its active state in the protein sample and is used to predict the plant seed trait.

In another preferred embodiment, the present invention relates to a method of predicting a plant seed trait by determining the presence of a target protein in its active state in a protein sample derived from a plant seed or plant seed lot, the method comprising:

(i) providing the protein sample;
(ii) contacting the protein sample with a chemical probe under conditions allowing the formation of a conjugate of the target protein and the chemical probe, wherein the chemical probe comprises a warhead being able to covalently attach to an amino acid residue in or nearby an active site of the target protein, and further comprises a reporter tag and a linker between the warhead and the reporter tag, and
(iii) using the reporter tag to detect the conjugate of step (ii), wherein detection of the conjugate indicates the presence of the target protein in its active state in the protein sample and is used to predict the plant seed trait.

As will be described hereinafter, each of the warhead, the linker, and the reporter tag of each chemical probe may be independently selected to provide different target specificities. Each of these components of such a chemical probe is described in detail below.

The "warheads" of the chemical probes can be selected from natural or unnatural moieties, including a variety of chemical moieties, such as synthetic compounds, naturally occurring compounds, e.g. amino acids, nucleotides, sugars, lipids, vitamins, enzyme cofactors and carbohydrates, and synthetic analogs thereof, which are readily available in a large variety of compounds. Some of the warheads can target specific classes of proteins, whereas other warheads can be reactive to a wide range of proteins (so called "non-directed warhead").

In general, the chemical probes as used herein comprise three different types of warheads: 1) the directed warhead, which irreversibly reacts with proteins from intended classes: 2) the photoaffinity warhead, which contains a reversible binding group and a photoreactive group, which provides a covalent bond between the probe and the target

protein upon a light pulse: and 3) the non-directed warhead. The latter is a mildly reactive chemical moiety that is not designed to label particular proteins, but scans the proteome for reactive moieties on proteins. These non-directed probes appear to label the active sites of proteins frequently.

Hence, the term "warhead" as used herein thus refers to the portion of a chemical probe that can be directed to and binds to a target protein in its active state. Preferably, said binding is a covalent binding. The warhead comprises a reactive group and an optional binding group. The reactive group refers to one or more chemical groups within a chemical probe that (preferably covalently) bind to or nearby the active site of a target protein. The reactive group may, by its very structure, be directed to the active site of a target protein. Optionally, a separate binding group may be provided in such probes. The binding group refers to a chemical group that is conjugated to the reactive group or associated with the linker, and provides enhanced binding affinity for protein targets and/or changes the binding profile of the warhead. The binding group may be a single atom, an ion or a free radical, and is preferably less than 1 kilodalton in mass.

Exemplary reactive groups as used in a chemical probe include an alkylating agent, acylating agent, ketone, aldehyde, sulphonate or a phosphorylating agent. Examples of particular reative groups used in a chemical probe include, but are not limited to fluorophosphonyl, fluorophosphoryl, fluorosulfonyl, alpha-haloketones or aldehydes or their ketals or acetals, respectively, alpha-haloacyls, nitriles, sulfonate alkyl or aryl thiols, iodoacetylamide group, maleimides, sulfonyl halides and esters, isocyanates, isothiocyanantes, tetrafluorophenyl esters, N-hydroxysuccinimidyl esters, acid halides, acid anhydrides, unsaturated carbonyls, alkynes, hydroxamates, alphahalomethylhydroxamates, aziridines, epoxides, or arsenates and their oxides. In this context, sulfonyl groups may include sulfonates, sulfates, sulfinates, sulfamates, etc., in effect, any reactive functionality having a sulfur group bonded to two oxygen atoms. In this context, epoxides may include aliphatic, aralkyl, cycloaliphatic and spiro epoxides.

In a preferred embodiment of the present invention, the warhead of the chemical probe comprises a reactive group which is selected from the group consisting of epoxides, fluorophosphonates, acyloxymethylketones, epoxyketone, vinyl sulphone, and acylphosphates.

In a further preferred embodiment of the present invention, the warhead of the chemical probe comprises a compound selected from the group consisting of brefeldin A, monensin, wortmannin, K-252a, H7, LY294002, staurosporine, genistein, calyculin A, okadaic acid, cantharidin, oryzalin, jasplakinolide, latrunculin B, taxol, paclitaxel, benzothiadiazole (BTH), U73122, E-64, AEBSF/pefablock, MG132, amanitin, cycloheximide, telomestatin, tunicamycin, cyclosporine A, paclobutrazol, uniconazole, mevinolin, lovastatin, naphthylphthalamic acid (NPA), 2,3,5-triiodobenzoic acid, nigericin, valinomycin, verapamil, dibutyryl cAMP, N-ethylmaleimide (NEM), forskolin, W7, A23187, leptomycin B, oligomycin, glibenclamide, quinidine, vinblastine, nifedipine, cytochalasin B, concanamycin A, bafilomycin, colchicines, BDM (2,3-dutanedione monoxime), bistheonellide A, amiprophosmethyl, probenecid, phalloidin, ML-7, actinomycin D, cerulenin, 2,6-dichloro-benzonitrile dichlobenil, isoxaben, atrazine, benoxacor, fenclorim, fluxofenim, fusiccocin, PD98059, U0126, 3,4-dephosphatin, dimethylthiourea, diphenylene iodonium (DPI), imidazole, tiron, thaxtomin A, phenylarsine oxide (PAO), and fluorophosphonate (FP).

One of the most effective classes of activity-based probes currently available for monitoring serine proteases are the reporter-tagged fluorophosphonate (FP) probes. These probes contain an FP group coupled to a reporter tag (e.g., rhodamine, biotin) by an alkyl or poly(ethylene glycol) linker and serve as broad spectrum profiling tools for serine proteases in biological samples. Most preferably, the warhead of the chemical probes used in the present invention comprises a fluorophosphonate (FP).

As described above, the chemical probes used in the present invention comprise a warhead, linked via a linker to a reporter tag. The term "linker" as used herein refers to a bond or chain of atoms used to link one moiety to another, serving as a covalent linkage between two or more moieties. The linker, which potentially can be as short as a covalent bond, is preferred to be other than a bond. Since in many cases, the synthetic strategy will be able to include a functionalized site for linking, the functionality can be taken advantage of in choosing the linker (or linking moiety). The choice of linker moiety has been shown to alter the specificity of a chemical probe (see, e.g., Kidd et al., Biochemistry (2001) 40: 4005-15). For example, an alkylene linker moiety and a linker moiety comprising a repeating alkyleneoxy structure (polyethylene glycols, or"PEG"), have distinct specificities and provide distinct protein profiles. Thus, one of skill in the art can select the linker moiety of the chemical probe in order to provide additional specificity of the probe for a particular protein or protein class.

Exemplary linkers as used in the chemical probes include, among others, ethers, polyethers, diamines, ether diamines, polyether diamines, amides, polyamides, polythioethers, disulfides, silyl ethers, alkyl or alkenyl chains (straight chain or branched and portions of which may be cyclic), aryl, diaryl or alkyl-aryl groups, having from 0 to 3 sites of aliphatic unsaturation. Preferably, the linker as used in a chemical probe is not an amino acid or an oligopeptide. If used, they will preferably employ amino acids of from 2-3 carbon atoms, i.e. glycine and alanine. Aryl groups in linker moieties can contain one or more heteroatoms (e.g., N, O or S atoms). The linker moieties, when other than a bond, will have from about 1 to 60-atoms, usually 1 to 30 atoms, where the atoms include C, N, O, S, P, etc., particularly C, N and O, and will generally have from about 1 to 12 carbon atoms and from about 0 to 8, usually 0 to 6 heteroatoms. The number of atoms referred to above are exclusive of hydrogen in referring to the number of atoms in a group, unless indicated

otherwise.

Exemplary linkers as used in the chemical probes can be varied widely depending on their function, including alkyleneoxy and polyalkyleneoxy groups, where alkylene is of from 2-3 carbon atoms, methylene and polymethylene, polyamide, polyester, and the like, where individual monomers will generally be of from 1 to 6, more usually 1 to 4 carbon atoms. The oligomers will generally have from about 1 to 10, more usually 1 to 8 monomeric units. The monomeric units can be amino acids, both naturally occurring and synthetic, oligonucleotides, both naturally occurring and synthetic, condensation polymer monomeric units and combinations thereof.

The term "reporter tag" as used herein refers to a molecule that can be used to detect and/or capture the chemical probe in combination with any other moieties that are bound strongly to the reporter tag, so as to be retained in the process of the reaction of the reactive group comprised by the warhead of the chemical probe with the active target protein. The reporter tag may be added to the warhead-linker moiety combination after reaction with the target protein, to form the complete chemical probe. For this purpose, the warhead-linker moiety combination will include a chemically reactive functionality, normally not found in proteins, that will react with a reciprocal functionality on the ligand, e.g. photoactivated groups, such as diazo bisulfites, etc. The warhead-linker moiety is then reacted with the reporter tag to complete the chemical probe.

The reporter tag portion thus can permit capture of the conjugate of the target protein and the chemical probe. The reporter tag may be displaced from the conjugate by addition of a displacing reporter tag, which may be free reporter tag or a derivative of the reporter tag, or by changing solvent (e.g., solvent type or pH) or temperature or the linker may be cleaved chemically, enzymatically, thermally or photochemically to release the isolated materials (see discussion of the linker, above).

Preferred reporter tags as used in the chemical probes include fluorescent moieties, radioactive moieties, electrochemical moieties, affinity moieties, and reactive moieties.

The term "fluorescent moiety" (also "fluorescent label") as used herein refers to a reporter tag that can be excited by electromagnetic radiation, and that emits electromagnetic radiation in response in an amount sufficient to be detected in an assay. The skilled person will understand that a fluorescent moiety absorbs and emits over a number of wavelengths, referred to as an "absorbance spectrum" and an "emission spectrum", respectively. A fluorescent moiety will exhibit a peak emission wavelength that is a longer wavelength than its peak absorbance wavelength. The term "peak" refers to the highest point in the absorbance or emission spectrum.

The fluorescent moiety may be varied widely depending upon the protocol to be used, the number of different probes employed in the same assay, whether a single or plurality of lanes are used in the electrophoresis, the availability of excitation and detection devices, and the like. For the most part, the fluorescent moieties that are employed as reporter tags will absorb in the ultraviolet, infrared, and/or most preferably in the visible range and emit in the ultraviolet, infrared, and/or most preferably in the visible range. Absorption will generally be in the range of about 250 to 750 nm and emission will generally be in the range of about 350 to 800nm.

Preferred fluorescent moieties include acridine dyes, cyanine dyes, fluorone dyes, oxazin dyes, phenanthridine dyes and rhodamine dyes. Particularly preferred fluorescent moieties include rhodamine dyes.

In a further particularly preferred embodiment the fluorescent labels FITC, Fluorescein, Fluorescein-5-EX, 5-SFX, Rhodamine Green-X, BodipyFL-X, Cy2, Cy2-OSu, Fluor X, 5 (6) TAMRA-X, Bodipy TMR-X, Rhodamine, Rhodamine Red-X, Texas Red, Texas Red-X, Bodipy TR-X, , Cy3-OSu, Cy3.5-OSu, Cy5, Cy5-Osu, Alexa fluors, Dylight fluors and/or Cy5.5-OSu may be used. These labels may be used either individually or in groups in any combination. Further suitable fluorescent moieties are known in the art, see e.g. The Handbook-A Guide to Fluorescent Probes and Labeling Technologies, Molecular Probes, 2005, which is incorporated herein by reference (or a more recent version thereof such as that published on *http://www.invitrogen.com/site/us/en/home/References/Molecula/-Probes- The-Handbook.html*).

The term "radioactive moiety" as used herein refers to a reporter tag containing a radioisotope (which is an isotope that has an unstable nucleus and that stabilizes itself by spontaneously emitting energy and particles). The main difference between the probe containing a radioactive moiety and the normal probe is that the radioactive moiety continually gives off radiation that can be detected with a Geiger counter or some other type of radiation detection instrument, such as a Phosphoimager. Suitable radioisotopes are commonly known in the art, e.g. $^{3}H$, $^{14}C$, $^{32}P$, $^{33}P$, $^{35}S$ or $^{125}I$.

The term "affinity moiety" as used herein refers to a reporter tag that is similar in structure to a particular substrate for a specific enzyme. Examples of such affinity moieties include biotin, digoxigenin, ssDNA, dsDNA, polypeptides, metal chelates and saccharides. Preferably, a chemical probe comprising such an affinity moiety is biotinylated. The term "biotinylated" as used herein means that said probe is covalently attached to the molecule biotin. Biotin remains the most commonly used reporter tag because of its ability to provide both a gel-based method of detection and a method for purification of labeled enzymes on streptavidin-agarose beads. The biotin-streptavidin bond is one of the strongest known non-covalent interactions with an association constant of $10^{15}$ $M^{-1}$ allowing for quantitative binding of low abundance biotinylated enzymes.

Hence, biotin facilitates detection by simple western blot approaches using a reporter avidin molecule in place

of the standard secondary antibody. Fluorescent and radioactive moieties can be visualized by direct scanning of gels with a fluorescent scanner/phosphoimager such as the Typhoon scanner from Amersham Biosciences. Fluorescent and radioactive moieties have several advantages over other reporter moieties. They are typically faster to use because they involve minimal time and handling. They also are more sensitive and have a greater dynamic range, especially when using the recently developed fluorescent labels such as the Alexa Fluors from Molecular Probes. Finally, they have the advantage of allowing multiplexing of samples based on non-overlapping excitation/emission spectra of the labels. This allows the skilled person to use probes with different colored fluorescent labels in different experiments and readily obtain all results on a single gel.

The term "electrochemical moiety" as used herein refers to a probe relying on a redox-active signal from the reporter tag, which changes upon the interaction of the target protein. Suitable electrochemical moieties are commonly known in the art.

Preferably, the electrochemical moiety is an enzymatic label. The term "enzymatic label" relates to labels, which comprise enzymatic activities. A typical, preferred example is the horseradish peroxidase enzyme (HRP) and the alkaline phosphatase (AP), which may be tethered or attached to the chemical probe. This enzyme complex subsequently may catalyze the conversion of a suitable substrate, e.g. a chemiluminescent substrate into a sensitized reagent in the vicinity of the complex which ultimatly lead to the emission of light or production of a color reaction. In particular, enhanced chemiluminescence, which may be used in this context, allows detection of minute quantities of labeled molecules.

The term "reactive moiety" as used herein refers to a reporter tag which contains a chemiluminescent label or a bioluminescent label. Furthermore, the term may also refer to a "bioorthogonal label" (e.g., using "click chemistry").

The term "chemiluminescent label" relates to a label which is capable of emitting light (luminescence) with a limited emission of heat as the result of a chemical reaction. Preferably, the term relates to luminol, cyalume, oxalyl chloride, TMAE (tetrakis (dimethylamino) ethylene), pyragallol, lucigenin, acridinumester or dioxetane.

The term "bio luminescent label" relates to a label, which is capable of emitting light due to a biochemical reaction. Typically, the term refers to the production of light due to the reaction of a luciferin and a luciferase. In such a reaction scheme, the luciferase catalyzes the oxidation of luciferin resulting in light and an inactive oxyluciferin. For example, a probe used in the present invention may be linked to a luciferase. Alternatively, the probe may also be labeled with luciferin. The luciferin and the luciferase as well as a co-factor such as oxygen, may be bound together to form a single photoprotein. This molecule may be linked to a chemical probe according to the present invention. A light emission may be triggered when a particular compound is present, e.g. a specific type of ion, preferably calcium. Examples of luciferin to be used in the context of the present invention include bacterial luciferin, dinoflagellate luciferin, vargulin, coelenterazine and firefly luciferin.

Alternatively, chemical probes used in the present invention can also be labeled or combined with fluorescent polypeptides, e.g. green fluorescent protein (GFP) as well as derivates thereof known to the person skilled in the art. These labels may be used either individually or in groups in any combination.

Chemical probes used in the present invention can also comprise a "click chemistry label" (or "bioorthogonal label"). Click chemistry describes a class of chemical reactions that use bioorthogonal or biologically unique moieties to label and detect a molecule of interest using a two-step procedure. For example, the two-step click reaction involves a copper-catalyzed triazole formation from an azide and an alkyne. The azide and alkyne moieties can be used interchangeably; either one can be used to tag the chemical probe, while the other is used for subsequent detection. The azides and alkynes are biologically unique, inert, stable, and extremely small. Click chemistry (or bioorthogonal chemistry) can be used when methods such as direct labeling or the use of antibodies are not applicable or efficient. The click chemistry label is small enough that tagged probes are acceptable substrates for the target proteins. The small size of click detection molecules allows them to easily penetrate complex samples with only mild permeabilization required. A wide variety of azide-or alkyne-containing dyes, haptens, and biomolecules for use in such reactions are commonly known in the art. A number of bioorthogonal reactions have been successfully developed that have shown excellent biocompatibility and selectivity in living systems (see, for example, R.K.V. Lim et al., Chem. Commun. 2010, 46: 1589-1600; which is incorporated herein by reference).

As described above, the fundamental building blocks of chemical probes according to the present invention are small-molecule compounds (i.e. the reactive group) that label, e.g., a given enzyme or enzymes. Ideal probes would target a large, but manageable, number of enzymes (tens to hundreds). This level of target promiscuity must, however, be counterbalanced by limited cross-reactivity with other proteins in the (sub-)proteome. Preferably, the probes achieve a desired level of intraclass coverage and minimal extraclass cross-reactivity by displaying a combination of reactive and binding groups that target conserved mechanistic or structural features in enzyme active sites.

In a preferred embodiment of the present invention, the warhead of the chemical probe comprises a reactive group directed to the active site of the target protein and an optional binding group, wherein

(i) the reactive group comprises an electrophilic/nucleophilic atom that is susceptible to attack by an active-site nucleophilic/electrophilic residue of the target protein (so called "classic probe");

(ii) upon reaction of an active-site residue of the target protein with the reactive group, an electrophilic/nucleophilic atom in the reactive group is provided, which electrophilic/nucleophilic atom is susceptible to attack by a non-catalytic nucleophilic/electrophilic residue of the target protein (so called "suicide probe");
(iii) upon addition of chemicals or UV light, a reactive center comprised by the reactive group is activated, which reactive center is susceptible to attack by an active-site nucleophilic or electrophilic residue of the target protein (so called "photo-affinity probe"); or
(iv) the reactive group is a "non-directed probe" (such as an alkylating agent).

In a particularly preferred embodiment of the present invention, the warhead of the chemical probe comprises a reactive group directed to the active site of the target protein and an optional binding group, wherein

(i) the reactive group comprises an electrophilic carbon atom that is susceptible to attack by an active-site nucleophilic residue of the target protein;
(ii) upon reaction of an active-site residue of the target protein with the reactive group, an electrophilic carbon atom in the reactive group is provided, which electrophilic carbon atom is susceptible to attack by a non-catalytic nucleophilic residue of the target protein;
(iii) upon addition of chemicals or UV light, a reactive center comprised by the reactive group is activated, which reactive center is susceptible to attack by an active-site nucleophilic or electrophilic residue of the target protein; or
(iv) the reactive group is an alkylating agent.

In general, the chemical probes according to the present invention includes four different classes of reactive groups, wherein the first two classes are probes based on true mechanism-based or suicide inhibitors and require active target enzymes to react with the probe.

In the case of the mechanism-based probes (i.e. the first class of probes), the key nucleophile is the catalytic residue of the enzyme normally involved in attack of a substrate. This type of chemical probe can therefore be made selective based on knowledge of catalytic mechanism and is often tailored to the reactivity of the specific nucleophilic atom used by the enzyme class. Examples of reactive groups in chemical probes include peptide acyloxymethyl ketones and epoxides that, inter alia, have been shown to efficiently and selectively label cysteine proteases. These reactive groups make use of an electrophilic atom that is susceptible to attack by the active-site nucleophile of the target protein (e.g. the enzyme). In the case of an epoxide probe, the result is the formation of a covalent bond between the active-site thiol residue and the electrophilic carbon in the epoxide ring. In theory, any reactive group that mimics a substrate and has an electrophilic atom could be used to covalently modify the active-site residue of a target protein that uses a nucleophilic attack mechanism. E.g., many enzyme classes utilize a nucleophilic attack mechanism at some point during the process of catalysis.

The second class of probes contain a "masked electrophile" that is uncovered after the probe functions as substrate for the target enzyme. The unmasked electrophile is able to react with nearby, non-catalytic, nucleophilic residues in the active site. Two examples of such reactive groups are sulbactam, a β-lactamase inhibitor used in combination with other antibiotics to combat bacterial antibiotic resistance, and DFPP, a probe that has been used to alkylate protein phosphatases. The proposed mechanism of action against phosphatases for DFPP involves initial attack by the active-site nucleophile on the phosphotyrosine mimetic resulting in dephosphorylation of the probe. This leads to production of a reactive quinone methide that can react with a nucleophilic side-chain, lysine or cysteine for example, found in the active site, resulting in irreversible alkylation of the enzyme. The potential drawback of this class of probes, i.e. it can lead to diffusion of unmasked electrophiles from the active-site pocket and alkylation of other sites on the target enzyme or other proteins that carry nucleophilic residues on their surface, can be overcome by adding specificity elements to the probes (i.e. an optional binding group, see above) in order to increase its affinity for the active site of the enzyme and keep the unmasked electrophile bound long enough for specific alkylation of the desired enzyme to occur.

The third class of probes are affinity alkylating probes containing affinity-based labeling groups that require only a strong nucleophile or electrophile in the vicinity of the active-site pocket and do not require the enzyme to be fully active. Probes carrying this class of reactive group must rely on the selectivity of the probe scaffold to direct modification to specific enzyme/protein classes. Preferably, such reactive groups are substrate analogs that contain a reactive center that is susceptible to attack by an electrophile or nucleophile in the active site of the target protein or that can be activated through the subsequent addition of chemicals or UV light.

The fourth class of reactive groups used for probe design contains non-specific alkylating groups that react with targets based only on the intrinsic reactivity of a specific amino acid residue such as cysteine. Chemical probes containing such reactive groups are also called "non-directed probes". Examples of such probes include sulfonate-ester probes (see, for example, G.C. Adam et al., Nature Biotechnology 2002, 20: 805-809).

As part of the inventive methods, compound libraries can be employed comprising chemical probes having affinity for one or more target proteins, wherein each chemical probe comprises a warhead being able to irreversibly

bind to an amino acid residue in or nearby an active site of the respective target protein, and further comprises a reporter tag and a linker between the warhead and the reporter tag.

The chemical libraries employed in the present invention contain a plurality of chemical probes, where the individual members each contain a reporter tag. The methods of the invention can employ such chemical probes e.g. for identifying suitable target proteins, i.e. molecular markers, in a protein sample comprising a mixture of proteins, which target proteins can be used for the prediction of a plant seed trait. The probes employed in the inventive methods can be both unselective (non-biased) or class-selective. Thus, the chemical libraries can contain chemical probes specific for a particular group of related proteins, and can contain one or a mixture of chemical probes, depending on the specificity of the probes and the variety in the group or groups of related target proteins to be assayed. Alternatively, chemical libraries can be employed to screen a protein sample for several plant seed traits.

In the context of the present invention, the term "chemical library" means a collection of chemical probes in which the probes are composed of one or more types of sub-units, including so-called "warheads". A "chemical library" employed in the inventive methods will comprise at least 2 chemical probes, rarely less than about 5 probes, usually at least about 10 probes, frequently will have about 50 probes or more, usually fewer than about 1,000 members, more usually fewer than about 500 members.

The term "plant" as used herein refers to living organisms belonging to the kingdom Plantae. Generally, they include familiar organisms such as trees, herbs, bushes, grasses, vines, ferns, mosses, and green algae. In particular, the present invention is directed to the spermatophytes (also known as phanerogams) comprising those plants that produce seeds. They are a subset of the embryophytes or land plants. The living spermatophytes form five groups: cycads, a subtropical and tropical group of plants with a large crown of compound leaves and a stout trunk; Ginkgo, a single living species of tree; conifers, cone-bearing trees and shrubs; gnetophytes, woody plants in the genera Gnetum, Welwitschia, and Ephedra; and angiosperms, the flowering plants, a large group including many familiar plants in a wide variety of habitats.

In the context of the present invention, a "plant seed" denotes a small embryonic plant enclosed in a covering called the seed coat. It is the product of the ripened ovule of gymnosperm and angiosperm plants which occurs after fertilization and some growth within the mother plant. The formation of the seed completes the process of reproduction in seed plants (started with the development of flowers and pollination), with the embryo developed from the zygote and the seed coat from the integuments of the ovule.

Generally, the term "plant seed" as used herein denotes anything that can be sown. Seeds are produced in several related groups of plants, and their manner of production distinguishes the angiosperms ("enclosed seeds") from the gymnosperms ("naked seeds"). Angiosperm seeds are produced in a hard or fleshy structure called a fruit that encloses the seeds. In gymnosperms, no special structure develops to enclose the seeds, which begin their development "naked" on the bracts of cones. However, the seeds do become covered by the cone scales as they develop in some species of conifer.

A typical plant seed as described herein includes three basic parts: (1) an embryo, (2) a supply of nutrients for the embryo, and (3) a seed coat. In addition to the three basic seed parts, some seeds have an appendage on the seed coat such an aril (as in yew and nutmeg) or an elaiosome (as in Corydalis) or hairs (as in cotton). The embryo is an immature plant from which a new plant will grow under proper conditions. Within the seed, there usually is a store of nutrients for the seedling that will grow from the embryo. The form of the stored nutrition varies depending on the kind of plant. The seed coat (or testa) develops from the tissue, the integument, originally surrounding the ovule. The seed coat in the mature seed can be a paper-thin layer (e.g. peanut) or something more substantial (e.g. thick and hard in honey locust and coconut). The seed coat helps protect the embryo from mechanical injury and from drying out.

Plant seeds can be very diverse in size. The dust-like orchid seeds are the smallest with about one million seeds per gram; they are often embryonic seeds with immature embryos and no significant energy reserves. At over 20 kg, the largest seed is the coco de mer. Plants that produce smaller seeds can generate many more seeds per flower, while plants with larger seeds invest more resources into those seeds and normally produce fewer seeds. Small seeds are quicker to ripen and can be dispersed sooner, so fall blooming plants often have small seeds. Many annual plants produce great quantities of smaller seeds; this helps to ensure that at least a few will end in a favorable place for growth. Herbaceous perennials and woody plants often have larger seeds, they can produce seeds over many years, and larger seeds have more energy reserves for germination and seedling growth and produce larger, more established seedlings after germination.

In a preferred embodiment, the plant seed is derived from a vegetable or a field crop. In the context of the present invention, the term "vegetable" denotes an edible plant or part of a plant other than a sweet fruit or seed. A "field crop" as used herein denotes a non-animal species or variety that is grown to be harvested as food, livestock fodder, fuel or for any other economic purpose and includes maize (corn), wheat, rice, soybeans, hay, potatoes and cotton.

In another preferred embodiment of the present invention, the plant seed is derived from a plant selected from the group consisting of: Tomato (*Solanum lycopersicum*), Melon (*Cucumis melo*), Pepper (*Capsicum annuum*), Lettuce (*Lactuca sativa*), Eggplant (*Solanum melongena*), Cucumber (*Cucumis sativus*), Squash (*Cucurbita pepo*), Watermelon

(*Citrullus lanatus*), Chicory (*Cichorium spp.*), Radish (*Raphanus sativus*), *Brassica spp.*, *Allium spp.*, Carrot (*Daucus carota*), Bean( *Phaseolus vulgaris*, *Vicia faba*), Pea (*Pisum sativum*), Parsley (*Petroselinum crispum*), Basil (*Ocimum basilicum*), Fennel (*Foeniculum vulgare*), Celery (*Apium graveolens*), Culin cardoon (*Cynara cardunculus*), Spinach (*Spinacia oleracea*), Corn salad (*Valerianella locusta*), Corn (*Zea mais*), Canola (*Brassica napus*), Sugar beet (*Beta vulgaris*), Potato (*Solanum tuberosum*), Wheat (*Triticum spp.*), Barley (*Hordeaum spp.*), Cotton (*Gossypium hirsutum*), Soybean (*Glycine max*), Rice (*Oryza sativa*), Flax (*Linum Usitatissimum*), Tobacco (*Nicotiana*), Pea (*Pisum sativum*), Sunflower (*Helianthus annuus*), Sorghum (*Sorghum vulgare*), Peanut (*Arachis hypogaea*), Oat (*Avena sativa*), Rye (*Secale cereale*), Lucerne *(Medicago sativa)* and Hemp (*Cannabis sativa*).

In a particularly preferred embodiment of the present invention, the plant seed is derived from a plant selected from the group consisting of: Tomato (*Solanum lycopersicum*), Melon (*Cucumis melo*), Pepper (*Capsicum annuum*), Lettuce (*Lactuca sativa*), Eggplant (*Solanum melongena*), Cucumber (*Cucumis sativus*), Squash (*Cucurbita pepo*), Watermelon (*Citrullus lanatus*), Corn (*Zea mais*), Canola (*Brassica napus*), Sugar beet (*Beta vulgaris*), Potato (*Solanum tuberosum*), Wheat (*Triticum spp.*), Barley (*Hordeaum spp.*), and Cotton (*Gossypium hirsutum*).

In another preferred embodiment of the present invention, the plant seed is derived from a vegetable selected from the group consisting of: Tomato (*Solanum lycopersicum*), Melon (*Cucumis melo*), Pepper (*Capsicum annuum*), Lettuce (*Lactuca sativa*), Eggplant (*Solanum melongena*), Cucumber (*Cucumis sativus*), Squash (*Cucurbita pepo*), and Watermelon (*Citrullus lanatus*), Chicory (*Cichorium spp.*), Radish (*Raphanus sativus*), *Brassica spp., Allium spp.,* Carrot (*Daucus carota*), Bean( *Phaseolus vulgaris*, *Vicia faba*), Pea *(Pisum sativum),* Parsley (*Petroselinum crispum*), Basil (*Ocimum basilicum*), Fennel (*Foeniculum vulgare*), Celery (*Apium graveolens*), Culin cardoon (*Cynara cardunculus*), Spinach (*Spinacia oleracea*) and Corn salad (*Valerianella locusta*).

Even more preferably, the plant seed is derived from a vegetable selected from the group consisting of: Tomato (*Solanum lycopersicum*), Melon (*Cucumis melo*), Pepper (*Capsicum annuum*), Lettuce (*Lactuca sativa*), Eggplant (*Solanum melongena*), Cucumber (*Cucumis sativus*), Squash (*Cucurbita pepo*), and Watermelon (*Citrullus lanatus*).

In another preferred embodiment of the present invention, the plant seed is derived from a field crop selected from the group consisting of: Corn (*Zea mais*), Canola (*Brassica napus*), Sugar beet (*Beta vulgaris*), Potato (*Solanum tuberosum*), Wheat (*Triticum spp.*), Barley *(Hordeaum spp.),* and Cotton *(Gossypium hirsutum),* Soybean (G*lycine max*), Rice (*Oryza sativa*), Flax (*Linum Usitatissimum*), Tobacco (*Nicotiana*), Pea (*Pisum sativum*), Sunflower (*Helianthus annuus*), Sorghum (*Sorghum vulgare*), Peanut (*Arachis hypogaea*), Oat (*Avena sativa*), Rye (*Secale cereale*), Lucerne *(Medicago sativa)*, and Hemp (C*annabis sativa*).

Even more preferably, the plant seed is derived from a field crop selected from the group consisting of: Corn (*Zea mais),* Canola (*Brassica napus*), Sugar beet (*Beta vulgaris*), Potato (*Solanum tuberosum*), Wheat (*Triticum spp.*), Barley (*Hordeaum spp.*), and Cotton (*Gossypium hirsutum*).

Most preferably, the plant seed is derived from Tomato *(Solanum lycopersicum).*

In the context of the present invention, a "plant seed trait" denotes a distinct variant of a phenotypic character of a plant seed that may be inherited, environmentally determined or somewhere in between. Hence, a trait may be any single feature or quantifiable measurement of a plant seed and is the final product of many molecular and biochemical processes (e.g., including a change in enzyme activity).

In a preferred embodiment of the present invention, the plant seed trait is selected from the group consisting of seed development, maturation, germination, germination rate, dormancy, storage deterioration, seed viability, sanitary state and vigour.

In the context of the present invention, "seed development" denotes a trait which is connected with the development phase of seeds. The seed, which is an embryo with two points of growth (one of which forms the stems the other the roots) is enclosed in a seed coat with some food reserves. Angiosperm seeds consist of three genetically distinct constituents: (1) the embryo formed from the zygote, (2) the endosperm, which is normally triploid, (3) the seed coat from tissue derived from the maternal tissue of the ovule. In angiosperms, the process of seed development begins with double fertilization and involves the fusion of the egg and sperm nuclei into a zygote. The second part of this process is the fusion of the polar nuclei with a second sperm cell nucleus, thus forming a primary endosperm. Right after fertilization, the zygote is mostly inactive but the primary endosperm divides rapidly to form the endosperm tissue. This tissue becomes the food that the young plant will consume until the roots have developed after germination or it develops into a hard seed coat. The seed coat forms from the two integuments or outer layers of cells of the ovule, which derive from tissue from the mother plant, the inner integument forms the tegmen and the outer forms the testa. When the seed coat forms from only one layer it is also called the testa, though not all such testa are homologous from one species to the next. In gymnosperms, the two sperm cells transferred from the pollen do not develop seed by double fertilization but one sperm nucleus unites with the egg nucleus and the other sperm is not used.

In the context of the present invention, "maturation" denotes a trait which is connected with seed maturation. Seed maturation is an important phase of seed development during which embryo growth ceases, storage products accumulate, the protective tegument differentiates and tolerance to desiccation develops, leading to seed dormancy. The spatial and temporal regulation of all these processes requires the concerted action of several signaling pathways

that integrate information from genetic programs, and both hormonal and metabolic signals.

In the context of the present invention, "germination" denotes trait which is connected with a process by which a seed embryo develops into a seedling (i.e. germination). Generally, germination involves the reactivation of the metabolic pathways that lead to growth and the emergence of the radicle or seed root and plumule or shoot. Three fundamental conditions must exist before germination can occur: (1) The embryo must be alive, called seed viability; (2) any dormancy requirements that prevent germination must be overcome; (3) the proper environmental conditions must exist for germination.

The term "seed viability" as used herein is the ability of the embryo to germinate and is affected by a number of different conditions. Some plants do not produce seeds that have functional complete embryos or the seed may have no embryo at all, often called empty seeds. Predators and pathogens can damage or kill the seed while it is still in the fruit or after it is dispersed. Environmental conditions like flooding or heat can kill the seed before or during germination. The age of the seed affects its health and germination ability: since the seed has a living embryo, over time cells die and cannot be replaced. Some seeds can live for a long time before germination, while others can only survive for a short period after dispersal before they die.

The term "germination percentage" as used herein is the proportion of seeds that germinate from all seeds subject to the right conditions for growth. The "germination rate" as used herein is the length of time it takes for the seeds to germinate. Germination percentages and rates are affected by seed viability, dormancy and environmental effects that impact on the seed and seedling. In agriculture and horticulture quality seeds have high viability, measured by germination percentage plus the rate of germination. This is given as a percent of germination over a certain amount of time, 90% germination in 20 days, for example. Environmental conditions effecting seed germination include: water, oxygen, temperature and light.

The term "dormancy" as used herein denotes a trait connected with seed dormancy which is defined as a seed failing to germinate under environmental conditions optimal for germination, normally when the environment is at a suitable temperature with proper soil moisture. This true dormancy or innate dormancy is therefore caused by conditions within the seed that prevent germination. Thus seed dormancy is a state of the seed, not of the environment. Induced dormancy, enforced dormancy or seed quiescence occurs when a seed fails to germinate because the external environmental conditions are inappropriate for germination, mostly in response to conditions being too dark or light, too cold or hot, or too dry. Many plants produce seeds with varying degrees of dormancy, and different seeds from the same fruit can have different degrees of dormancy. It's possible to have seeds with no dormancy if they are dispersed right away and do not dry (if the seeds dry they go into physiological dormancy). There is great variation amongst plants and a dormant seed can still be a viable seed even though the germination rate might be very low.

The term "seed dormancy" as used herein includes exogenous dormancy, endogenous dormancy, photodormancy and thermodormancy.

Exogenous dormancy is caused by conditions outside the embryo including: Physical dormancy (seeds are impermeable to water) and chemical dormancy (a chemical prevents germination).

Endogenous dormancy is caused by conditions within the embryo itself, including: Morphological dormancy (germination is prevented due to morphological characteristics of the embryo), morphophysiological dormancy seeds (underdeveloped embryos), physiological dormancy (the embryo can, due to physiological causes, not generate enough power to break through the seed coat, endosperm or other covering structures), combinational dormancy (the seed or fruit coat is impermeable to water and the embryo has physiological dormancy), and secondary dormancy (caused by conditions after the seed has been dispersed and occurs in some seeds when non-dormant seed is exposed to conditions that are not favorable to germination, very often high temperatures).

Furthermore, the following types of seed dormancy do not involve seed dormancy strictly spoken as lack of germination is prevented by the environment not by characteristics of the seed itself: Photodormancy (seed sensitivity to the presence or absence of light) and thermodormancy (seed sensitivity to heat or cold).

In the context of the present invention, "storage deterioration" (also called "longevity" or "shelf life") denotes deteriorative changes occurring with time that increase the seed's vulnerability to external challenges and decrease the ability of the seed to survive.

In the context of the present invention, "sanitary state" denotes a distinct variant of a phenotypic character of a plant seed that is connected with seed infection with pests (herbivores) and/or pathogens (microorganisms) that cause plant diseases. Diseases of plants can be caused by fungi, bacteria, viruses, mollicutes, and nematodes. Fungi are eukaryotic, spore-bearing, heterotrophic organisms that produce extracellular enzymes to break down plant or animal products to small molecules (for example, sugars and amino acids), which they absorb as nutrients. Bacteria, although differing from fungi in being unicellular prokaryotic organisms, also cause disease by extracellular digestion of plant tissues. The viruses that cause plant diseases are also often carried by insects and other pests, as well as by grafting and on cutting tools, machinery, or in seed. Plant pathogenic nematodes are small wormlike animals that live in soil and feed on plant roots by piercing the cells with a needlelike structure called a stylet through which they suck up the cell contents.

In the context of the present invention, "vigour" denotes a measure of the quality of seed, and involves the viability of the seed, the germination percentage, germination rate and the strength of the seedlings produced.

In a particularly preferred embodiment of the present invention, the plant seed trait is selected from the group consisting of germination and vigour. Most preferably, the plant seed trait is seed viability or germination percentage.

According to the present invention, a plant seed trait can be predicated by determining the presence of a target protein in its active state in a protein sample derived from a single plant seed or a plant seed lot. The term "active state" as used herein generally refers to a protein is in its native conformation, which protein is able to interact with an entity that it normally interacts with, e. g. enzyme with substrate and cofactor, receptor with ligand, etc.

In a preferred embodiment of the present invention, the target protein in its active state is an enzyme or a receptor in its active form. It is particularly preferred that the target protein is an enzyme. Even more preferably, the target protein is selected from the group consisting of proteases of all catalytic classes, serine hydrolases, kinases, phosphatases, glycosidases, cytochrome P450s, acyltransferases and histone deacetylases.

In another preferred embodiment of the present invention, the target protein is selected from the group consisting of enzymes that affect seed storage deterioration, enzymes that have a role in the endosperm weakening, enzymes involved in the hormone metabolism, enzymes that have a role in the mobilization of seed storage reserves, and deshydrogenases.

Enzymes that affect seed storage deterioration are known in the art and include, for example, catalase, glutathion reductase, superoxyde dismutase, dehydroascorbate reductase and ascorbate peroxydase. Enzymes that have a role in the endosperm weakening are known in the art and include, for example, glucanase, galacturonase, galactosidase and mannanase. Enzymes involved in the hormone metabolism (especially in the abscisic acid metabolism and gibberellins metabolism) are known in the art and include, for example,gibberelin oxidases, abscisic acid aldehyde oxidases, and abscisic acid hydroxylases. Enzymes that have a role in the mobilization of seed storage reserves are known in the art and include, for example, hydro lases.

Hence, in another particularly preferred embodiment of the present invention, the target protein is selected from the group consisting of proteases of all catalytic classes, serine hydrolases, kinases, phosphatases, glycosidases, cytochrome P450s, acyltransferases, histone deacetylases, catalase, glutathion reductase, superoxyde dismutase, dehydroascorbate reductase, ascorbate peroxydase, glucanase, galacturonase, galactosidase, mannanase, gibberelin oxidases, abscisic acid aldehyde oxidases, abscisic acid hydroxylases, hydrolases and deshydrogenases.

As discussed above, the chemical probe according to the present invention comprises a warhead being able to (preferably covalently) attach to an amino acid residue in or nearby an active site of the target protein. An "active site" of a target protein, as used herein, refers to the specific area on the surface of a protein, e.g., an enzyme which reacts with the probe. In the context of the present invention, the active site denotes the site(s) of an enzyme where the substrate and/or a cofactor bind, and where the substrate and cofactor undergo a catalytic reaction.

In the context of the present invention, the term "enzyme" denotes a protein contained in a plant seed that catalyzes (i.e., increase the rates of) biochemical reactions. Almost all processes in a biological cell need enzymes to occur at significant rates. Since enzymes are selective for their substrates and speed up only a few reactions from among many possibilities, the set of enzymes made in a cell determines which metabolic pathways occur in that cell.

The term "serine hydrolases" as used herein denotes a large and diverse enzyme class that includes proteases, peptidases, lipases, esterases, and amidases. Serine hydro lases are united by a common catalytic mechanism that involves activation of a conserved serine nucleophile for attack on a substrate ester/thioester/amide bond to form an acyl-enzyme intermediate, followed by water-catalyzed hydrolysis of this intermediate to liberate the product.

In a preferred embodiment of the present invention, the target protein is a serine hydrolase. The greatly enhanced nucleophilicity of the catalytic serine renders it susceptible to covalent modification by many types of electrophiles, including fluorophosphonates (FPs) and aryl phosphonates, sulfonyl fluorides, and carbamates. Preferably, the chemical probe then comprises one of these electrophiles, even more preferably a fluorophosphonate (FP). Exemplary fluorophosphonate probes comprising a reporter tag ("TAG") are shown below (formulae 1 and 2):

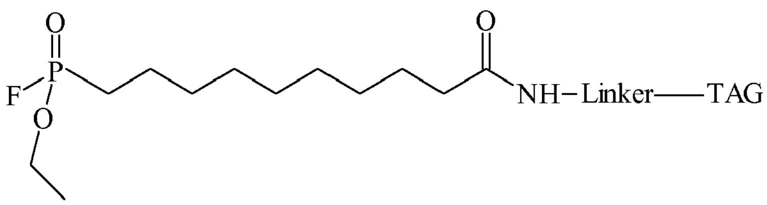

(1)

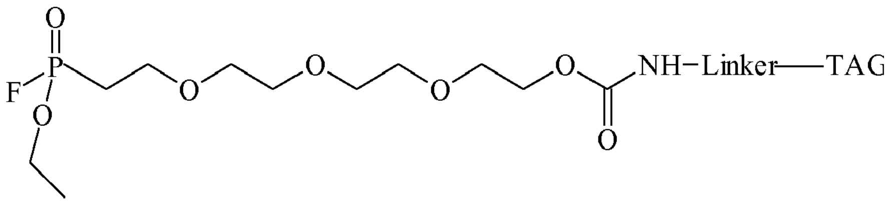

(2)

The term "proteases" as used herein denotes any enzyme that conducts proteolysis, that is, begins protein catabolism by hydrolysis of the peptide bonds that link amino acids together in the polypeptide chain forming the protein. Exemplary proteases include serine proteases, threonine proteases, cysteine proteases, aspartate proteases, metalloproteases, and glutamic acid proteases.

In a further preferred embodiment of the present invention, the target protein is a cysteine protease. Cysteine proteases are a huge enzyme class whose members perform myriad critical functions in prokaryotic and eukaryotic organisms. The distinct catalytic mechanisms employed by SHs and cysteine proteases, however, make them susceptible to inactivation by different electrophilic chemotypes. Preferably, the chemical probe then comprises one of these chemotypes, including epoxides, vinyl sulfones, diazomethyl ketones, α-halo ketones, and acyloxymethyl ketones. An exemplary epoxide probe comprising a reporter tag (TAG) is shown below (formula 3):

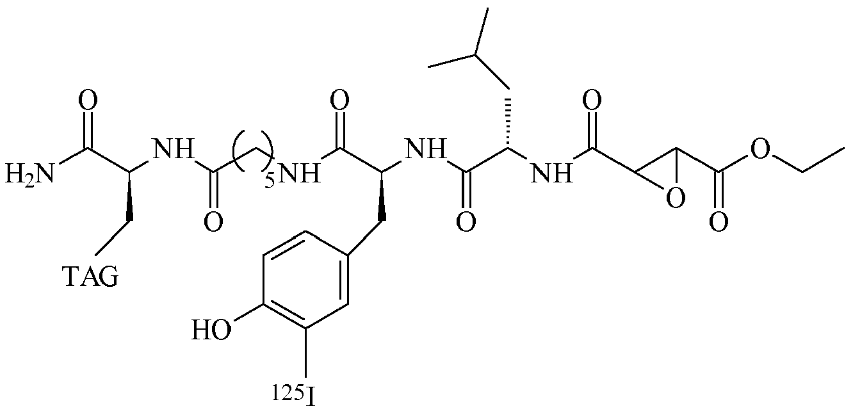

(3)

In a further preferred embodiment of the present invention, the target protein is a metalloprotease including proteases, peptidases, and deacetylases. Unlike serine or cysteine hydrolases, which use enzyme-bound nucleophiles for catalysis, the metallohydrolases accomplish substrate hydrolysis by a zinc-activated water molecule. This difference in mechanism makes necessary an alternative design of electrophilic chemical probes for metallohydrolases. As an alternative strategy, photoreactive variants of reversible inhibitors of metallohydrolases can be introduced into chemical probes. These photoreactive probes thus achieve target selectivity through binding affinity, rather than "mechanism-based" reactivity. Covalent labeling is accomplished by exposure to UV light, which induces the photoreactive group to modify proteins in close spatial proximity to the probes.

In a further preferred embodiment of the present invention, the target protein is a histone deacetylase. The reversible acetylation of lysine residues on histone proteins plays a critical role in transcriptional activation and repression. Lysine deacetylation is catalyzed by a family of enzymes, the histone deacetylases, which function as parts of larger protein complexes. Class I and II histone deacetylases are zinc-dependent metallohydrolases. An exemplary probe (click chemistry-based) is shown below (formula 4):

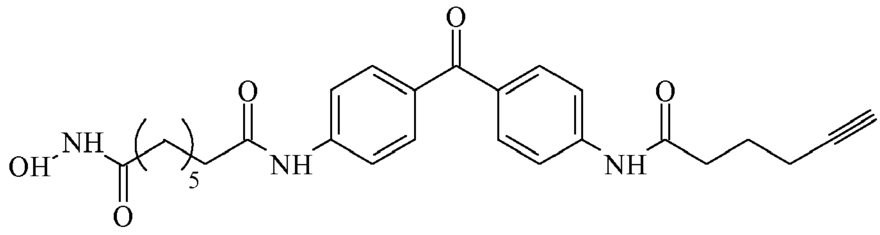

(4)

In a further preferred embodiment of the present invention, the target protein is a kinase. Despite the sequence and structural diversity within the kinome, protein kinases all recognize a common substrate, ATP, the key phosphate donor in the phosphoryl transfer to protein or peptide substrates. Not unexpectedly, the ATP binding site is the region with the highest sequence homology among kinases and contains two consensus motifs that serve as signature sequences for nearly all protein kinases. In the case of the serine and cysteine hydrolases discussed above, the eponymous amino acid residues are hypernucleophilic and are catalytically essential to the formation of covalent enzyme-bound intermediates leading to products. Such nucleophilic residues are ideally suited for specific covalent modification by electrophilic probes. Kinases catalyze phosphate transfer from ATP to substrate by a direct transfer mechanism that does not involve covalent enzyme intermediates. Thus, the kinase active site does not contain any unusually nucleophilic residues. Conserved active-site lysines do, however, present a possible target for chemical probes. Generally, chemical probes targeting kinases can be based on 5'-p-fluorosulfonylbenzoyladenosine, can use ATP or ADP as a scaffold, or can covalently bind to the lysine residue in the active site.

Preferably, the chemical probe then is based on ADP or ATP (see, for example, M.P. Patricelli, Biochemistry 2007, 46: 350-358; which is incorporated herein by reference). Exemplary probes comprising an ATP (BHAcATP) or ADP (BHAcADP) binding group, an acyl phosphate reactive group, and a biotin tag are shown below (formulae 5 and 6):

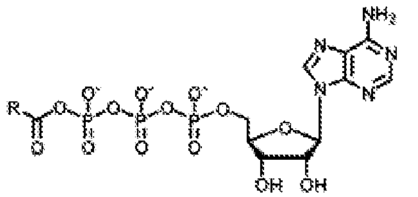

BHAcATP

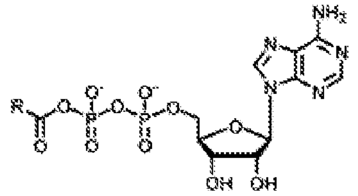

BHAcADP

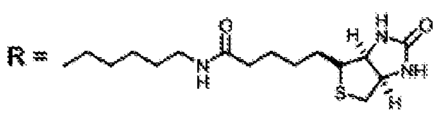

(5) (6)

In a further preferred embodiment of the present invention, the target protein is a phosphatase. Protein phosphorylation is a reversible posttranslational modification. Two major classes of phosphatases, the serine/threonine and tyrosine phosphatases, are responsible for removing phosphate groups from proteins. Serine/threonine phosphatases are metaldependent enzymes that function as part of multisubunit complexes. Several natural products have been identified that potently inhibit serine/threonine phosphatases, including the cyanobacterial hepatotoxin microcystin, which covalently modifies a noncatalytic cysteine residue in the active sites of these enzymes. Preferably, the chemical probe then comprises one of these inhibitors.

In a further preferred embodiment of the present invention, the target protein is a glycosidase. Glycosidases are a large class of enzymes found in all branches of life that play key roles in cellular metabolism and in the regulation of carbohydrate modifications to lipids and proteins.

In a further preferred embodiment of the present invention, the target protein is a cytochrome P450. The cytochrome P450 (P450) monooxygenase enzyme family metabolizes a large number of endogenous signaling molecules, xenobiotics, and drugs. The activity of P450 enzymes is regulated by multiple factors in vivo: membrane composition and localization; protein-binding partners, posttranslational modification, endogenous concentrations of cofactors (e.g., NAPDH), and regulatory enzymes (e.g., NADPH-P450 reductases).

In a further preferred embodiment of the present invention, the target protein is an acyltransferase which is a type of transferase enzyme which acts upon acyl groups. Examples of acyltransferases include: Glyceronephosphate O-acyltransferase and lecithin-cholesterol acyltransferase.

In a preferred embodiment of the present invention, the chemical probe comprises a moiety selected from the group consisting of epoxides (e.g., for the detection of papain-like cysteine proteases), fluorophosphonates (e.g., for the detection of serine hydrolases and acyltransferases), acyloxymethylketones (e.g., for the detection of legumains), epoxyketone (e.g., for the detection of proteasome), vinyl sulphone (e.g., for the detection of cystein proteases and/or proteasome) and acylphosphates (e.g., for the detection ofkinases).

Suitable chemical probes for detecting activity of one or more of the enzyme classes described above are known in the art (see, e.g., B.F. Cravatt et al., Annual Review of Biochemistry 2008, 77: 383-414; which is incorporated herein by reference).

The methods described herein find use for the most part with protein samples, which may have been subject to processing before reaction with the chemical probes. The term "protein sample", as used herein, intends a sample derived from plant seeds, preferably from a single plant seed. Examples of protein samples include proteins obtained from plant seeds, particularly as a protein extract, or the like.

Of particular interest are samples that are "complex protein mixtures". As used herein, this phrase refers to protein mixtures having at least about 20, more usually at least about 50, even 100 or more different proteins. An example of such a complex protein mixture is a proteome, as defined hereinafter. Complex protein mixtures may be obtained from seeds that are normal or abnormal in some particular traits such as dormancy, longevity, viability, vigour, or the like.

The term "proteome" as used herein refers to a complex protein mixture obtained from a plant seed. Preferred proteomes comprise at least about 5% of the total repertoire of proteins present in a protein sample, preferably at least about 10%, more preferably at least about 25%, even more preferably about 75%, and generally 90% or more, up to and including the entire repertoire of proteins obtainable from the protein sample. The proteome will be a mixture of proteins, generally having at least about 20 different proteins, usually at least about 50 different proteins and in most cases 100 different proteins or more.

In a preferred embodiment of the present invention, the protein sample is a protein extraction from a plant seed or a plant seed lot. Hence, the protein sample is provided by extracting the proteins from a single plant seed or from a plant seed lot.

In particular, proteomes from single or multiple seeds can be extracted in different ways. First of all, it is possible to extract the entire seed or only a part of it, e.g. the embryo or endosperm. Secondly, the protein sample can be derived from different subproteomes, e.g. soluble proteins, membrane proteins, different organelles (nucleus, mitochondria, etc.). Thirdly, the storage conditions of protein extracts can be varied (e.g. immediate use vs. frozen storage. Finally, the protein sample can be derived from different biochemical separations, e.g. using ion-exchange or MW filters. In the context of the present invention, the protein sample can be provided by applying any combination of the above-described ways.

Preferably, the protein sample is provided by grinding at least one plant seed in an extraction buffer and removing insoluble materials by centrifugation. The initial step of the process of providing the protein sample thus involves solubilizing proteinaceous material from the plant seed or plant seeds. This can be accomplished by homogenizing the seed or seeds using a suitable buffer. Preferred extractants are borate buffer solutions at concentrations of 5 to 500 mM (more preferably, of 10 to 100 mM, and most preferably of 40 to 60 mM) and pH of 7.0 to 10.0 (more preferably, of 7.0 to 9.0, and most preferably of 7.0 to 8.0). Preferably, extractions can be carried out at an ambient temperature of 24°C to 34°C, using a mechanical grinder. Subsequently, insoluble materials can be removed by centrifugation and the protein sample is provided by collecting the supernatant. Further suitable protein extraction methods are commonly known in the art.

After providing the protein sample, the protein sample is contacted with a chemical probe under conditions allowing the formation of a conjugate of the target protein and the chemical probe. Alternatively, the plant seed or plant seed lot is directly contacted with a chemical probe under conditions allowing the formation of a conjugate of the target protein and the chemical probe and the protein sample is subsequently provided. In preferred embodiments, chemical probe and reaction conditions are selected such that the relative ability of an active target protein to become labeled depends on the relative level of activity of that active target protein; the signal obtained from such labeling can be correlated to the activity of the active target proteins in the proteomic mixture. Alternatively, chemical probes can be used under conditions in which all active forms of an active target protein are labeled, regardless of the level of activity of the particular active target protein. For example, the time of reaction may be extended so that the labeling reaction goes substantially to completion; the signal obtained from such labeling will be unrelated to the activity of the active target proteins in the proteomic mixture.

Suitable reaction conditions allowing the formation of a conjugate of the target protein and the chemical probe are known to the person skilled in the art, and particularly include probe concentration, labeling time, buffer content (pH, salt, cofactors, redox, inhibitors), proteome concentration, reaction volume etc. Preferably, the reaction is carried out in a suitable buffer at pH of 4.0 to 9.0 and a salt concentration of 0 to 250 mM. Furthermore, it is preferred that the buffer has a redox potential (e.g. the buffer comprises DTT or the like). Preferably, the reaction volume is less than 50 $\mu$L. Preferably, the reaction is carried out for at least 15 minutes, most preferably for 60 minutes.

Following the labeling reaction, the reporter tag comprised by the probe is used to detect the conjugate of the active target protein and the chemical probe, wherein detection of the conjugate indicates the presence of the target protein in its active state in the protein sample. The detection of the conjugate of the active target protein and the chemical probe and/or measuring the protein level of the target protein may be carried out via any suitable detection technique known in the art.

In a preferred embodiment of the present invention, detection of the conjugate is accomplished, for example, by the separation of proteins from the sample on a polyacrylamide gel, followed by visualization of the reporter tag, e.g. by direct scanning of the gel with a fluorescent scanner such as the Typhoon scanner from Amersham Biosciences (if the reporter tag is a fluorescent moiety) or by direct scanning of the gel with a phosphoimager (if the reporter tag is a radioactive moiety) or by using a reporter avidin molecule in place of the standard secondary antibody (if the reporter tag is biotin). Alternatively, proteins contained in the protein sample can be separated by two-dimensional gel electrophoresis systems. Two-dimensional gel electrophoresis is well known in the art and typically involves iso-electric focusing along a first dimension followed by SDS-PAGE electrophoresis along a second dimension. The analysis of 2D SDS-PAGE gels can be performed by determining the intensity of protein spots on the gel (e.g. as described above), or can be performed using immune detection. In other embodiments, protein samples are analyzed by mass spectroscopy (e.g., if the reporter tag is biotin).

The conjugate may further be detected immunologically, e.g. by using a molecule specific for the reporter tag (if the reporter tag is an affinity moiety). The present invention also envisages the use of peptide affinity ligands like aptamers specific for the reporter tag.

Immunological tests which may be used in the context of the present invention, in particular for the detection purposes of the present invention, include, for example, competitive and non-competitive assay systems using techniques such as western blots, radioimmunoassay like RIA (radio-linked immunoassay), ELISA (enzyme linked immunosorbent assay), "sandwich" immunoassays, immunoprecipitation assays, precipitin reactions, gel diffusion precipitin reactions, immunodiffusion assays, agglutination assays, e.g. latex agglutination, complement-fixation assays, immunoradiometric assays, fluorescent immunoassays, e.g. FIA (fluorescence-linked immunoassay), chemiluminescence immunoassays, electrochemiluminescence immunoassay (ECLIA) and protein A immunoassays. Such assays are routine and well known to the person skilled in the art.

The conjugate may also be detected in methods involving histological or cell-biological procedures. Typically, visual techniques, such as light microscopy or immunofluoresence or UV microscopy, as well as flow cytometry or luminometry may be used.

Suitable visualization methods to detect the conjugate are known to the person skilled in the art. Typical methods to be used comprise fluorometric, luminometric and/or enzymatic techniques. Fluorescence is normally detected and/or quantified by exposing fluorescent labels to light of a specific wavelength and thereafter detecting and/or quantifying the emitted light of a specific wavelength. The presence of a luminescently tagged affinity ligand may be detected and/or quantified by luminescence developed during a chemical reaction. Detection of an enzymatic reaction is due to a color shift in the sample arising from chemical reaction.

Optionally the target proteins may be further assayed to identify the specific proteins to which the probes bound. In particular, isolated target proteins can be optionally characterized by mass spectrometric (MS) techniques (e.g., by in-gel digestion of target proteins or by standard on-bead digestion of probe-labeled target proteins after affinity purification). In particular, the sequence of isolated target proteins can be determined using tandem mass spectrometric techniques, and by application of sequence database searching techniques, the target protein from which a sequenced peptide originated can be identified.

Furthermore, following reaction of the protein sample with a probe, the resulting protein conjugates can be proteolytically digested to provide probe-labeled peptides. Mass spectrometry may be employed to identify one or more probe-labeled peptides by molecular weight and/or amino acid sequence. The sequence information derived from of the probe-labeled peptide(s) may be used to identify the target protein from which the peptide originally derived. Variations of these aspects can involve the comparison of two or more proteomes, e.g., with probes having different reporter tags, or, when analysis comprises mass spectrometry, having different isotopic compositions.

For example, the specific proteins to which the probes bound can be identified using the MudPIT technology (multidimensional protein identification technology), i.e. a non-gel approach for the identification of proteins from complex mixtures. This technique consists of a 2-dimensional chromatography separation, prior to electrospray mass spectrometry. By exploiting a peptide's unique physical properties of charge and hydrophobicity, complex mixtures can be separated prior to sequencing by tandem MS.

In a preferred embodiment of the present invention, the method further comprises separating the proteins contained in the protein sample by one or more means selected from the group consisting of liquid chromatography, electrophoretic separation, precipitation, immunocapture, microfluidic separation and mass spectrometry.

"Chromatography" is the collective term for a set of laboratory techniques for the separation of mixtures. It involves passing a mixture dissolved in a "mobile phase" through a stationary phase, which separates the proteins of interest from other proteins in the mixture based on differential partitioning between the mobile and stationary phases. In the context of the present invention, liquid chromatography may be preparative or analytical. The purpose of preparative chromatography is to separate the components of a mixture for further use (and is thus a form of purification). Analytical chromatography is done normally with smaller amounts of material and is for measuring the relative proportions of analytes in a mixture. The two techniques are not mutually exclusive.

"Electrophoretic separation" (or gel electrophoresis) is a technique used for the separation of protein molecules using an electric field applied to a gel matrix. Electrophoretic separation is usually performed for analytical purposes, but may be used as a preparative technique prior to use of other methods such as mass spectrometry for further characterization.

"Precipitation" is widely used in downstream processing of biological products, such as proteins. This operation serves to concentrate and fractionate the target protein from various contaminants. The underlying mechanism of precipitation is to alter the solvation potential of the solvent and thus lower the solubility of the solute by addition of a reagent.

In the context of the present invention, the term "immunocapture" denotes the use of capture antibodies which are able to isolate target proteins (e.g., large enzyme complexes in their fully intact, fully active states) from protein samples.

"Microfluidic separation" involves the use of microfluidic chip suitable for high efficiency continuous separation of liquid protein samples.

"Mass spectrometry" (MS) is an analytical technique for the determination of the elemental composition of a sample or molecule. It is also used for elucidating the chemical structures of molecules, such as peptides and other chemical compounds. The MS principle consists of ionizing chemical compounds to generate charged molecules or molecule fragments and measurement of their mass-to-charge ratios.

In another preferred embodiment of the present invention, the conjugate is separated from non-target proteins by means of liquid chromatography, electrophoretic separation and/or mass spectrometry. In the context of the present invention, the term "non-target proteins" denotes proteins comprised by the protein sample or the plant seed or plant seed lot which do not form conjugates upon contact with the chemical probe, i.e. the warhead comprised by the chemical probe does not attach to an amino acid residue of the non-target protein.

Subsequent to the detection step, the presence of the target protein in its active state in the protein sample is used to predict the plant seed trait. In this context, "presence of the target protein" includes the actual presence of the target protein in its active state in the protein sample, the actual absence of the target protein in its active state in the protein sample and any increase or decrease of the target protein level in its active state in the protein sample. Hence, in the context of the present invention, the term "detection" may refer to qualitative and/or quantitative detection of the target protein in its active state in the protein sample. For example, if the actual presence of the target protein in its active state in the protein sample can be used to predict a seed trait, it may be possible to screen a seed batch for the presence of said active target protein. For example, if the actual absence of the target protein in its active state in the protein sample can be used to predict a seed trait, it may be necessary to screen each single seed for the absence of said active target protein.

In a preferred embodiment of the present invention, the plant, the plant seed trait and the target protein are each selected from the preferred plants, preferred traits and preferred target proteins as listed in the table below, including any possible combination thereof:

| Preferred plant | Preferred trait | Preferred target protein |
|---|---|---|
| Tomato, Melon, Pepper, Lettuce, Eggplant, Cucumber, Squash, Watermelon, Chicory, Radish, *Brassica spp., Allium spp.,* Carrot, Bean, Pea, Parsley, Basil, Fennel, Celery, Culin cardoon, Spinach, Corn salad, Corn, Canola, Sugar beet, Potato, Wheat, | Seed development, maturation, germination, dormancy, storage deterioration, vigour, seed viability, germination percentage, sanitary state and germination rate. | Protease, serine hydrolase, kinase, phosphatase, glycosidase, cytochrome P450, acyltransferase and histone deacetylase, catalase, glutathion reductase, superoxyde dismutase, dehydroascorbate reductase, ascorbate peroxydase, glucanase, |

(continued)

| Preferred plant | Preferred trait | Preferred target protein |
|---|---|---|
| Barley, Cotton, Soybean, Rice, Flax, Tobacco, Pea, Sunflower, Sorghum, Peanut, Oat, Rye, Lucerne and Hemp. | | galacturonase, galactosidase, mannanase, gibberelin oxidase, abscisic acid aldehyde oxidase, abscisic acid hydroxylase , hydrolase and deshydrogenase. |

In a particularly preferred embodiment of the present invention, the plant, the plant seed trait and the target protein are each selected from the preferred plants, preferred traits and preferred target proteins as listed in the table below, including any possible combination thereof:

| Preferred plant | Preferred trait | Preferred target protein |
|---|---|---|
| Tomato, Melon, Pepper, Lettuce, Eggplant, Cucumber, Squash, Watermelon, Corn, Canola, Sugar beet, Potato, Wheat, Barley, and Cotton. | Seed development, maturation, germination, dormancy, storage deterioration, vigour, seed viability, germination percentage, sanitary state and germination rate. | Protease, serine hydrolase, kinase, phosphatase, glycosidase, cytochrome P450, acyltransferase and histone deacetylase. |

For example, in a particularly preferred embodiment, the present invention can relate to a method of predicting seed viability of a single plant seed, preferably from Tomato (*Solanum lycopersicum*), by determining the presence of a serine hydrolase in its active state in a protein sample derived from the plant seed, the method comprising:

(i) providing the protein sample;
(ii) contacting the protein sample with a chemical probe under conditions allowing the formation of a conjugate of the serine hydrolase and the chemical probe, wherein the chemical probe comprises a warhead being able to covalently attach to an amino acid residue in or nearby an active site of the serine hydrolase, and further comprises a reporter tag and a linker between the warhead and the reporter tag, and
(iii) using the reporter tag to detect the conjugate of step (ii), wherein detection of the conjugate indicates the presence of the serine hydrolase in its active state in the protein sample and is used to predict seed viability of the single plant seed.

The skilled person would know that the greatly enhanced nucleophilicity of the catalytic serine of the serine hydrolase of interest renders it susceptible to covalent modification by many types of electrophiles, including fluorophosphonates (FPs) and aryl phosphonates, sulfonyl fluorides, and carbamates. Preferably, the chemical probe then would comprise one of these electrophiles, even more preferably a fluorophosphonate (FP).

It is known in the art that certain plant proteins (in particular, enzymes) can be connected with particular plant traits. For example, WO 2005/054499 discloses that an L-isoaspartyl methyltransferase antibody can be used for determining the germination vigour and/or the storability of a seed batch. Further connections between seed traits and potential target proteins are known to the person skilled in the art. Suitable chemical probes for detecting activity of one or more of the preferred target proteins listed above are known in the art (see, e.g., B.F. Cravatt et al., Annual Review of Biochemistry 2008, 77: 383-414; which is incorporated herein by reference).

Comparative ABPP can be used to confirm that particular traits are connected with certain target proteins. This method concerns the comparison of protein activities in proteomes generated from various samples. The detected differential activities between samples can be used as a molecular marker associated with a physiological process, or uncover a protein activity that contributes to the process itself. This method can also be adapted for high-throughput analysis using automated extraction procedures and fluorescent probes. For example, it is possible to screen seed proteomes with different activity-based probes to identify markers that predict various seed traits. The molecular origin of the markers can be identified by mass spectrometry.

In some embodiments, the methods of the invention are performed in a high-throughput format. In a further embodiment, the invention relates to the use of a method, as described herein, for the prediction of a seed trait. In a further embodiment, the invention relates to the use of a chemical probe, as described herein, for the prediction of a seed trait.

While the above invention has been described with respect to some of its preferred embodiments, this is in no way to limit the scope of the invention. The person skilled in the art is clearly aware of further embodiments and alterations

to the previously described embodiments that are still within the scope of the present invention.

## EXAMPLES

### Example 1

Materials:

Activity-based probes (cf. Figure 6):

| | |
|---|---|
| VPE-R | for Vacuolar Processing Enzyme |
| βN3DCG-04 | for Papain-like cysteine proteases |
| MV151 | for the proteasome |
| FP-Rh | for Ser hydrolases |

Used tomato seeds: two seed lots of varieties A and B, with different viabilities:

A1 (92%); A3 (74%); B1 (94%); B3 (79%)

In general, seeds (one per reaction) were ground in eppendorf tube with water or labelling buffer. Subsequently, the protein extract was centrifuged and supernatant was taken for labeling. Labeling was performed for 1-2 hours with various fluorescent activity-based probes (see above) in various labeling buffers. Proteins were subsequently separated on protein gels. Fluorescently labeled proteins were detected by fluorescence scanning.

In particular, one tomato seed was homogenized in 100 μl 50 mM borate buffer pH7.6 using two steel beads in an eppendorf tube. Of the extract, 5 μl was taken into a 50 μl reaction volume containing 2 μM probe and buffer (1 mM DTT; 50 mM (NaOAc pH 5.5 or Tris pH 7.6)) and incubated for 1 hour (FP-Rh) or 3 hours (ßN3DCG-04 and MV151) at room temperature. 25 μl of a 4x protein gel-loading buffer containing was added, the samples were heated for 5 minutes at 95 °C and 10 μl sample was separated on polyacrylamide protein gels containing SDS. Protein gels were scanned for fluorescence using the Fuij FLA6000 scanner.

Figure 2 depicts an illustration of the application of a preferred embodiment of the invention. A general workflow of a probe analysis in parallel by using fluorescence gel electrophoresis is shown. A plant seed extract was labeled using four different chemical probes. Labeling was performed on seed proteins in two buffers:

A: 50 mM NaOAc, pH 5.5, 1 mM DTT
B: 50 mM Tris, pH 7.6, 1 mM DTT.

Four different chemical probes have been used to label the plant seed extract:

1: VPE-R (Vacuolar Processing Enzyme)
2: ßN3DCG-04 (Cys proteases)
3: MV 151 (proteasome) and
4: FP-Rh (Ser hydrolases).

The labeled protein samples were then used for the preparation of a fluorescence gel. Control lanes (-) depict unlabeled protein extract. Robust labelling was detected with probes 2,3 and 4.

### Example 2

Figure 3 depicts a fluorescence gel demonstrating that ABPP can be used to label different seed lots. Several plant seed extracts derived from different seed lots were labeled using the chemical probe FP-Rh (Ser hydrolases). Labeling was performed on seed proteins derived from seed lots A1, A3, B1 and B3. The labeled protein samples were then used for the preparation of a fluorescence gel. Control lanes (-) depict unlabeled protein extract. Robust labelling was detected for all seed lots.

**Example 3**

Figure 4 depicts a fluorescence gel demonstrating that differential protein activities can be detected upon seed hydratation. A plant seed extract derived from a dry seed (lane D) and a plant seed extract derived from a seed which was hydrated for 24 hours (lane I) was labeled using three different chemical probes. Labeling was performed on seed proteins in two buffers:

A: 50 mM NaOAc, pH 5.5, 1 mM DTT
B: 50 mM Tris, pH 7.6, 1 mM DTT.

Three different chemical probes have been used to label the plant seed extracts, respectively:

1: ßN3DCG-04 (Cys proteases)
2: MV 151 (proteasome) and
3: FP-Rh (Ser hydrolases).

The labeled protein samples were then used for the preparation of a fluorescence gel. Control lanes (-) depict unlabeled protein extract. The last lane depicts an additional 40 kDa Ser hydrolase activity upon hydratation.

**Example 4**

Figure 5 depicts a fluorescence gel demonstrating that the inventive method can be used to detect differences in enzyme activity between viable and non-viable seeds. Plant seed extracts derived from dead seeds (seed numbers 6, 24 and 41) and plant seed extracts derived from viable seeds (seed numbers 7, 14 and 35) were labeled using the chemical probe FP-Rh (Ser hydrolases). A further plant seed extract (seed number 13) derived from a seed which could not be classified as viable or non-viable. Control lanes (-) depict unlabeled protein extract. The labeled protein samples were then used for the preparation of a fluorescence gel. The lanes containing labeled protein extract depict an additional 53 kDa Ser hydrolase activity that correlates with seed viability.

The seed viability testing was performed as follows:

Seeds were longitudinally cut in two parts. Each part of the seed should contain embryo and testa tissues. One half of the seed was used for the labeling with probes. The second half was used for the viability test. The 2,3,5-triphenyl tetrazolium chloride (TTC) staining procedure is used for testing viability in seeds of different species. A solution of 1% TTC was made in water. Half seeds were placed in Eppendorf tubes with 100 μL TTC solution. Tubes were stored in darkness during 7 hours. Seeds were then briefly washed and observed with a binocular. Seeds with a red embryo (root + cotyledon) were considered viable. The other ones were considered as dead seeds.

**Claims**

**1.** Method of predicting a plant seed trait by determining the presence of a target protein in its active state in a protein sample derived from a plant seed or plant seed lot, the method comprising:

(i) providing the protein sample;
(ii) contacting the protein sample or the plant seed or plant seed lot with a chemical probe under conditions allowing the formation of a conjugate of the target protein and the chemical probe, wherein the chemical probe comprises a warhead being able to attach to an amino acid residue in or nearby an active site of the target protein, and further comprises a reporter tag and a linker between the warhead and the reporter tag, and
(iii) using the reporter tag to detect the conjugate of (ii), wherein detection of the conjugate indicates the presence of the target protein in its active state in the protein sample and is used to predict the plant seed trait.

**2.** Method according to any of the preceding claims, wherein the method further comprises separating the proteins contained in the protein sample by one or more means selected from the group consisting of liquid chromatography, electrophoretic separation, precipitation, immunocapture, microfluidic separation and mass spectrometry.

**3.** Method according to any of the preceding claims, wherein the conjugate is separated from non-target proteins by means of liquid chromatography, electrophoretic separation and/or mass spectrometry.

**4.** Method according to any of the preceding claims, wherein the method further comprises identifying the target protein

by means of mass spectrometry.

**5.** Method according any of the preceding claims, wherein the target protein in its active state is an enzyme or a receptor in its active form.

**6.** Method according any of the preceding claims, wherein the protein sample is a protein extraction from a plant seed or a plant seed lot.

**7.** Method according any of the preceding claims, wherein the protein sample is provided by grinding at least one plant seed in an extraction buffer and removing insoluble materials by centrifugation.

**8.** Method according any of the preceding claims, wherein the plant seed is derived from a vegetable or a field crop.

**9.** Method according any of the preceding claims, wherein the plant seed is derived from a plant selected from the group consisting of: Tomato (*Solanum lycopersicum*), Melon (*Cucumis melo*), Pepper (*Capsicum annuum*), Lettuce (*Lactuca sativa*), Eggplant (*Solanum melongena*), Cucumber (*Cucumis sativus*), Squash (*Cucurbita pepo*), Watermelon (*Citrullus lanatus*), Corn (*Zea mais*), Canola (*Brassica napus*), Sugar beet (*Beta vulgaris*), Potato (*Solanum tuberosum*), Wheat (*Triticum spp.*), Barley (*Hordeaum spp.*), and Cotton (*Gossypium hirsutum*).

**10.** Method according to any of the preceding claims, wherein the warhead of the chemical probe comprises a reactive group directed to the active site of the target protein and an optional binding group, wherein

(i) the reactive group comprises an electrophilic atom that is susceptible to attack by an active-site nucleophilic residue of the target protein;
(ii) upon reaction of an active-site nucleophilic residue of the target protein with the reactive group, an electrophilic atom in the reactive group is provided, which electrophilic atom is susceptible to attack by a non-catalytic nucleophilic residue of the target protein;
(iii) upon addition of chemicals or UV light, a reactive center comprised by the reactive group is activated, which reactive center is susceptible to attack by an active-site nucleophilic or electrophilic residue of the target protein; or
(iv) the reactive group is a non-directed probe.

**11.** Method according to any of the preceding claims, where the warhead of the chemical probe comprises a reactive group which is selected from the group consisting of epoxides, fluorophosphonases, acyloxymethylketones, epoxyketone, vinyl sulphone, and acylphosphates.

**12.** Method according to any of the preceding claims, where the reporter tag of the chemical probe is a detectable label selected from the group consisting of fluorescent moieties, radioactive moieties, electrochemical moieties, affinity moieties, and reactive moieties.

**13.** Method according to any of the preceding claims, where the conditions allowing the formation of a conjugate of the target protein and the chemical probe comprise contacting the protein sample with the chemical probe at a pH of between 4.0 and 9.0.

**14.** Method according to any of the preceding claims, wherein the plant seed trait is selected from the group consisting of seed development, maturation, germination, germination rate, dormancy, storage deterioration, seed viability, sanitary state and vigour.

**15.** Use of a chemical probe for the prediction of a plant seed trait, wherein the chemical probe comprises a warhead being able to attach to an amino acid residue in or nearby an active site of the target protein, and further comprises a reporter tag and a linker between the warhead and the reporter tag.

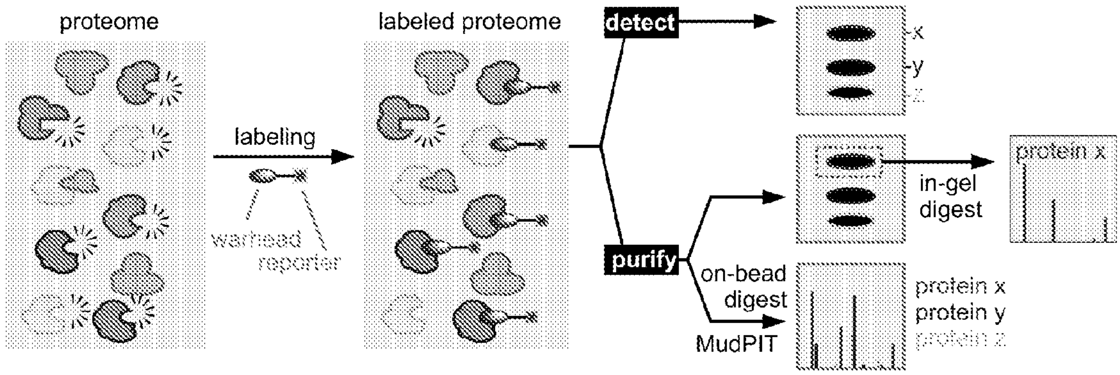
proteome
labeled proteome
labeling
warhead
reporter
detect
purify
x
y
z
in-gel
digest
protein x
on-bead
digest
MudPIT
protein x
protein y
protein z

**Figure 1**

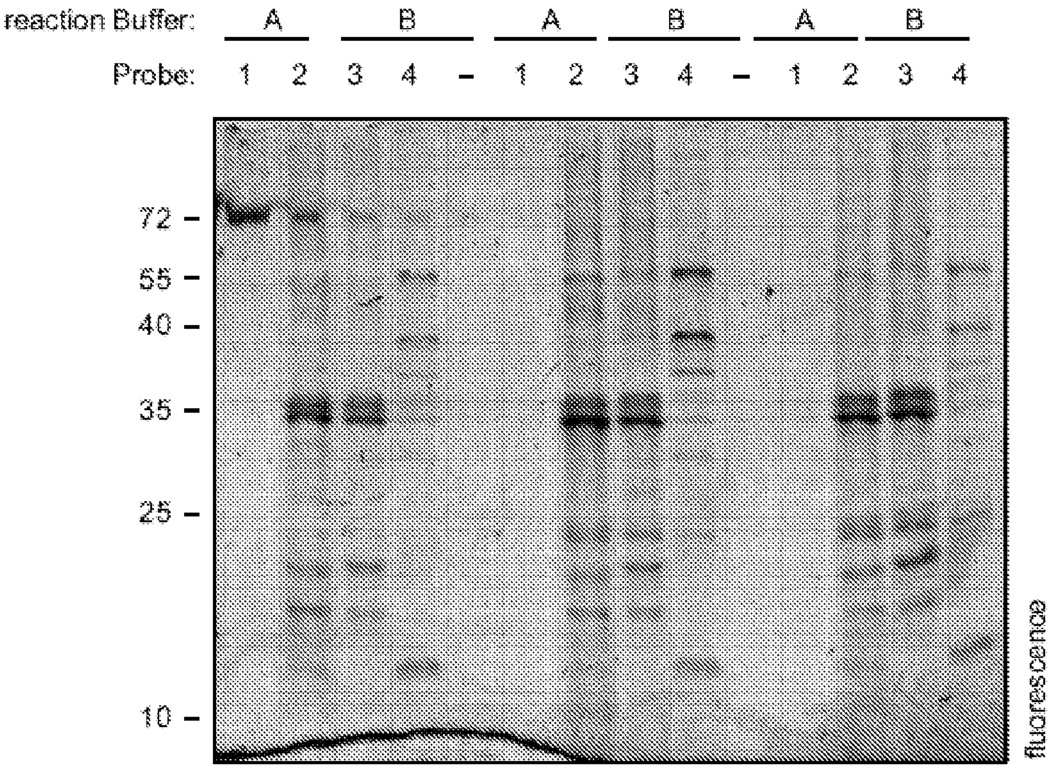
reaction Buffer: A B A B A B
Probe: 1 2 3 4 – 1 2 3 4 – 1 2 3 4
72
55
40
35
25
10
fluorescence

Figure 2

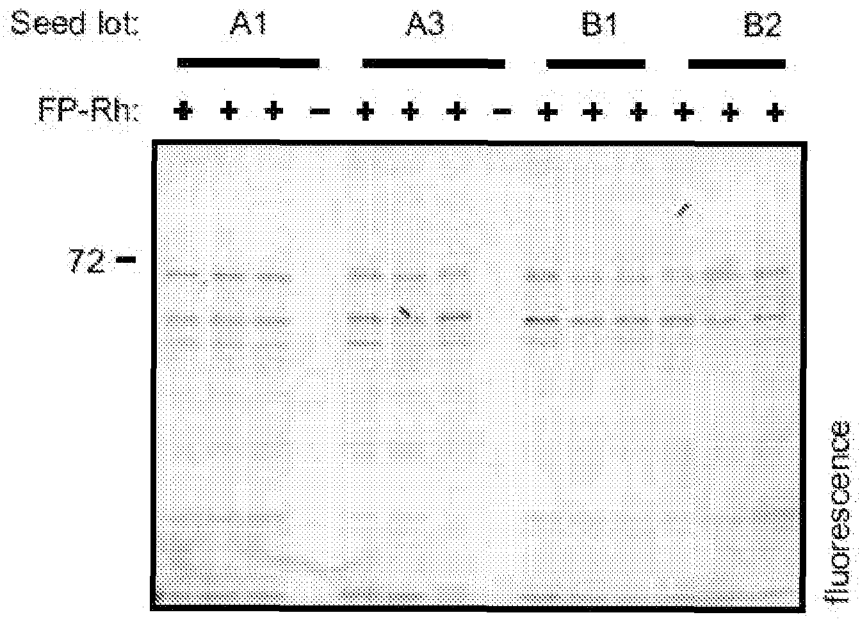


Figure 3

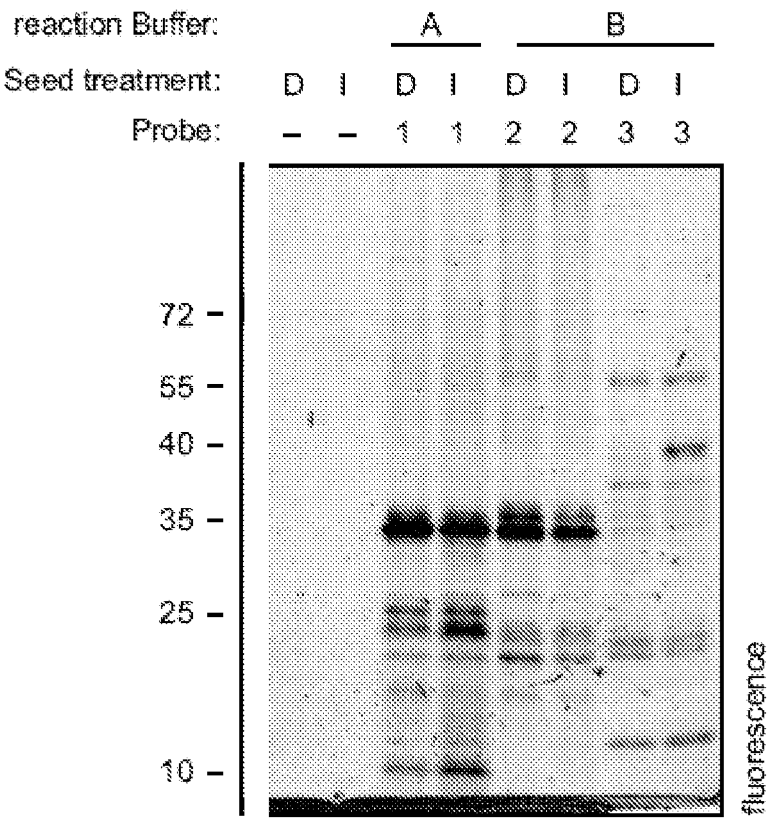
reaction Buffer:
A
B
Seed treatment:
D I D I D I D I
Probe:
– – 1 1 2 2 3 3
72
55
40
35
25
10
fluorescence

Figure 4

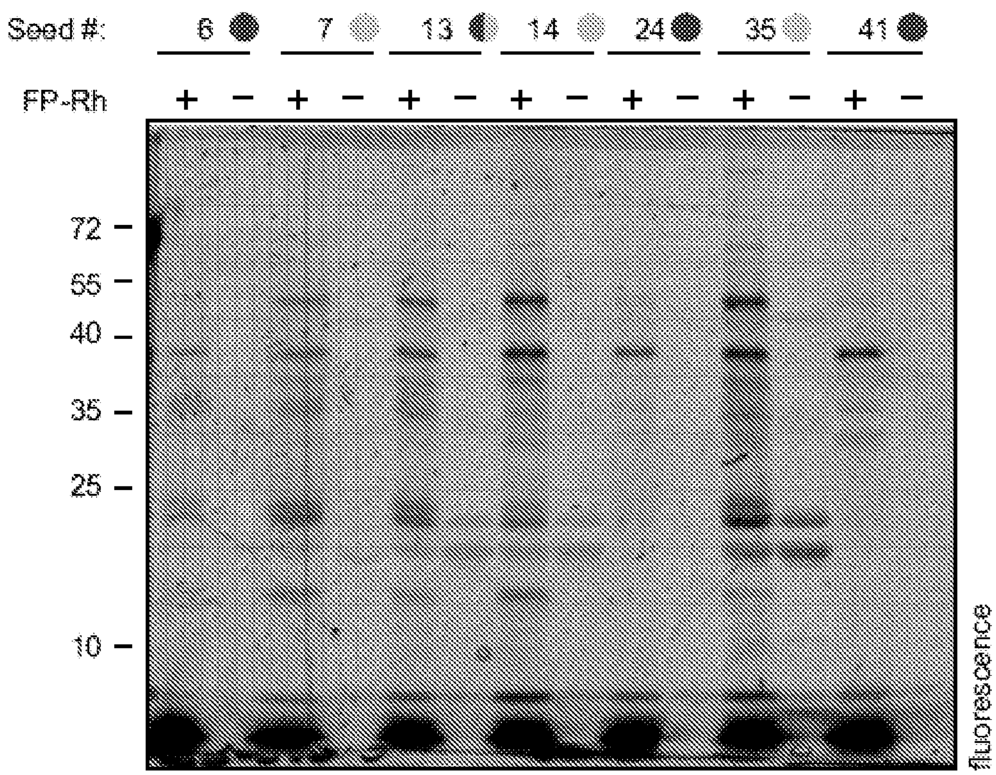


Figure 5

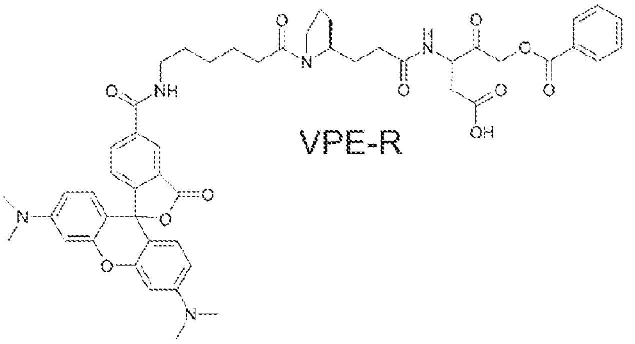

VPE-R

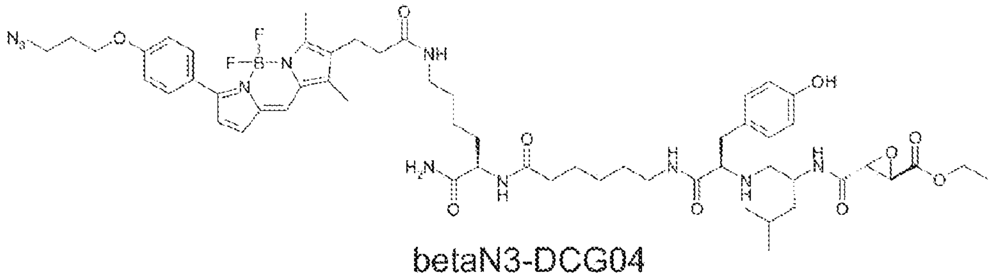

betaN3-DCG04

MV151

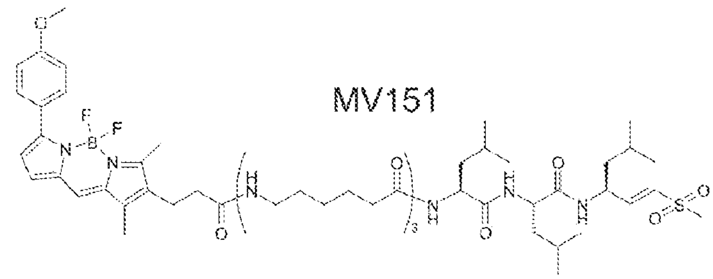

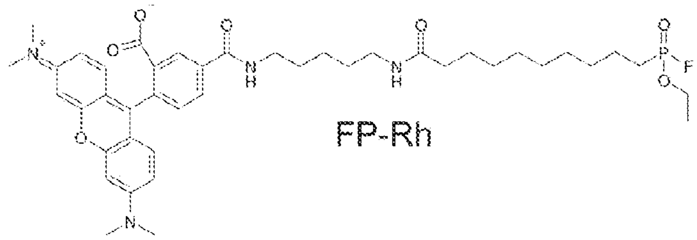

FP-Rh

**Figure 6**

Europäisches Patentamt
European Patent Office
Office européen des brevets

# EUROPEAN SEARCH REPORT

Application Number
EP 11 15 2509

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Christian Gu: "Activity-based protein profiling in plants", Inaugural-DissertationzurErlangung des Doktorgradesder Mathematisch-Naturwissenschaftlichen Fakultätder Universität zu Köln , 5 March 2010 (2010-03-05), XP002632203, Köln Retrieved from the Internet: URL:http://kups.ub.uni-koeln.de/3015/ [retrieved on 2011-04-08] * the whole document * * paragraph [2.3.4]; figures 3-5 * | 1-15 | INV. G01N33/68 |
| X,D | KOLODZIEJEK IZABELLA ET AL: "Mining the active proteome in plant science and biotechnology", CURRENT OPINION IN BIOTECHNOLOGY, vol. 21, no. 2, April 2010 (2010-04), pages 225-233, XP002632204, ISSN: 0958-1669 * the whole document * * page 228 * | 1-15 | TECHNICAL FIELDS SEARCHED (IPC): G01N |
| A | US 2007/036725 A1 (BOGYO MATTHEW S [US] ET AL) 15 February 2007 (2007-02-15) * the whole document * | 1-15 | |

The present search report has been drawn up for all claims

1

| Place of search | Date of completion of the search | Examiner |
|---|---|---|
| Munich | 11 April 2011 | Bayer, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 15 2509

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2011

| Patent document cited in search report | Publication date | Patent family member(s) | Publication date |
|---|---|---|---|
| US 2007036725 A1 | 15-02-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005054499 A


### Non-patent literature cited in the description


- **D.A. JEFFRY ; M. BOGYO.** *Current Opinion in Biotechnology,* 2003, vol. 14, 87-95
- **B.F. CRAVATT et al.** *Annual Review of Biochemistry,* 2008, vol. 77, 383-414
- **I. KOLODZIEJEK ; R.A.L. VAN DER HOORN.** *Current Opinion in Biotechnology,* 2010, vol. 21, 225-233
- **KIDD et al.** *Biochemistry,* 2001, vol. 40, 4005-15
- Handbook-A Guide to Fluorescent Probes and Labeling Technologies. Molecular Probes, 2005
- **R.K.V. LIM et al.** *Chem. Commun.,* 2010, vol. 46, 1589-1600
- **G.C. ADAM et al.** *Nature Biotechnology,* 2002, vol. 20, 805-809
- **M.P. PATRICELLI.** *Biochemistry,* 2007, vol. 46, 350-358